# EUROPÄISCHE PATENTSCHRIFT

(11) **EP 2 064 169 B1**
(45) Veröffentlichungstag und Bekanntmachung des Hinweises auf die Patenterteilung: **23.03.2016**
(21) Anmeldenummer: 07803136.6
(22) Anmeldetag: 31.08.2007
(51) Int. Cl.: C07C 29/17, C07C 29/56, C07C 29/80, C07C 35/12, C07C 35/17, B01J 31/02, B01J 31/14, B01J 31/40

(54) **RÜCKGEWINNUNG VON BIS(DIARYLPHENOL)-LIGANDEN BEI DER HERSTELLUNG VON ISOPULEGOL**
RECOVERY OF BIS(DIARYLPHENOL) LIGANDS DURING THE PRODUCTION OF ISOPULEGOL
RECUPERATION DE LIGANDS BIS(DIARYLPHENOL) LORS DE LA PRODUCTION D'ISOPULEGOL

(30) Priorität: 01.09.2006 EP 06120013
(43) Veröffentlichungstag der Anmeldung: 03.06.2009
(73) Patentinhaber: BASF SE, 67056 Ludwigshafen (DE)
(72) Erfinder: HEYDRICH, Gunnar, 67117 Limburgerhof (DE); GRALLA, Gabriele, 68161 Mannheim (DE); EBEL, Klaus, 68623 Lampertheim (DE)
(86) Internationale Anmeldenummer: PCT/EP2007/059149
(87) Internationale Veröffentlichungsnummer: WO 2008/025852

(56) Entgegenhaltungen:
- EP-A- 1 225 163
- WO-A-2006/056435
- WO-A-2006/069659
- WO-A-2006/092433

## Beschreibung

Die vorliegende Erfindung betrifft ein Verfahren zur Aufarbeitung eines Aluminium-haltigen Reaktionsprodukts aus der Herstellung von Isopulegol durch Cyclisierung von Citronellal in Gegenwart von Komplexverbindungen, umfassend wenigstens einen Liganden der Formel (I).

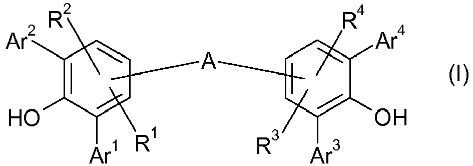

Darüber hinaus betrifft die Erfindung ein Verfahren zur Herstellung von Isopulegol sowie ein Verfahren zur Herstellung von Menthol.

Menthol stellt weltweit die mengenmäßig wichtigste Aromachemikalie dar. Der Bedarf an Menthol wird nach wie vor zu einem großen Teil durch Isolierung aus natürlichen Quellen gedeckt. Daneben existieren jedoch auch synthetische Zugänge zum Menthol, teils in racemischer Form, teils in Form des natürlichen Enantiomeren L-Menthol.

Ein wichtiges Zwischenprodukt zur Herstellung von racemischem wie optisch aktivem Menthol stellt Isopulegol dar, das üblicherweise durch cyclisierende Oxo-En-Reaktion von Citronellal in Gegenwart von Lewis-sauren Katalysatoren hergestellt wird und dabei in der Regel in Form von Gemischen der vier Diastereomere Isopulegol, iso-Isopulegol, neo-Isopulegol und neoiso-Isopulegol anfällt.

Als geeignete Katalysatoren zur Durchführung der vorstehend genannten Cyclisierung von Citronellal zu Isopulegol wurden sowohl heterogene Katalysatoren, wie SiO₂, Al₂O₃/SiO₂-, SiO₂/ZrO₂-, SiO₂/TiO₂-Mischkatalysatoren, Mordenite, Faujasite, Monmorillonite und Zeolithe - als auch homogene Katalysatoren, wie beispielsweise Sulfonsäuren oder Lewis-Säuren, wie beispielsweise SnCl₄, ZnCl₂ oder ZnBr₂ beschrieben.

Die EP-A 1 225 163 beschreibt die Cyclisierung von Citronellal zu Isopulegol in Gegenwart von Tris(2,6-diphenylphenol)-aluminium-Katalysatoren. Bei diesem Verfahren zur Cyclisierung von Citronellal zu Isopulegol werden teure und nur aufwändig herzustellende Katalysatorkomplexe eingesetzt. Nach dem beschriebenen, in homogener Phase durchzuführenden Verfahren wird der Katalysatorkomplex nach beendeter Reaktion hydrolysiert. Eine mögliche Wiedergewinnung und Wiedereinsetzbarkeit des dabei freigesetzten Liganden ist nicht beschrieben.

Demgegenüber beschreibt die WO2006/092433 Bis(diarylphenoxy)-Aluminium-Verbindungen, die durch Umsetzung eines Bis(diarylphenol)-Liganden der Formel (I) mit einer geeigneten Aluminiumverbindung erhältlich sind sowie Verfahren zur Herstellung von Isopulegol und Menthol in Gegenwart dieser Verbindungen. Dabei werden auch Verfahren offenbart, die eine Rückgewinnung der verwendeten Bis(diarylphenol)-Liganden der Formel (I) ermöglichen. Die Rückgewinnung erfolgt durch Kristallisation aus einem bei der destillativen Abtrennung von Isopulegol aus einem Reaktionsprodukt der Cyclisierung von Citronellal erhaltenen Sumpfprodukt. Eine solche Aufarbeitung führt jedoch, insbesondere bei einem kontinuierlichen Verfahren zur Herstellung von Isopulegol, zu Ausbeuten und Reinheiten, die nicht vollständig zufriedenstellend sind.

Aufgabe der vorliegenden Erfindung war es demnach, ein Verfahren bereitzustellen, das nach erfolgter Cyclisierung von Citronellal zu Isopulegol erlaubt, die eingesetzten Bis(diarylphenol)-Liganden mit verbesserter Reinheit und Ausbeute zurückzugewinnen und wiedereinzusetzen. Damit soll speziell ein kontinuierliches Verfahren mit guter Raum-Zeit-Ausbeute ermöglicht werden.

Überraschenderweise wurde nun gefunden, dass sich die eingesetzten Bis(diarylphenol)-Liganden nach erfolgter Cyclisierung von Citronellal durch destillative Auftrennung des Aluminium-haltigen Reaktionsprodukts unter Erhalt eines an Isopulegol angereicherten Kopfprodukts und eines an Isopulegol abgereicherten Sumpfprodukts, anschließendes Inkontaktbringen des an Isopulegol abgereicherten Sumpfprodukts mit einer wässrigen Base unter Erhalt einer Aluminium-haltigen wässrigen Phase und einer die Hauptmenge der Liganden der Formel (I) enthaltenden organischen Phase und anschließendes Abtrennen des Liganden der Formel (I), bevorzugt durch Kristallisation, aus der organischen Phase in verbesserter Reinheit und Ausbeute zurückgewinnen lässt.

Gegenstand der vorliegenden Erfindung ist somit ein Verfahren zur Aufarbeitung eines Aluminium-haltigen Reaktionsprodukts aus der Herstellung von Isopulegol durch Cyclisierung von Citronellal, enthaltend
i) Isopulegol,
ii) wenigstens einen Liganden der Formel (I), 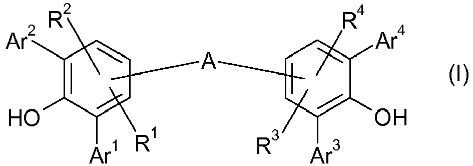
wobei
Ar¹, Ar², Ar³, Ar⁴ unabhängig voneinander ausgewählt sind unter C₆-C₁₅-Arylresten oder C₂-C₁₅-Heteroarylresten, die gegebenenfalls jeweils 1 bis 7 gleiche oder verschiedene Substituenten, ausgewählt unter C₁-C₆-Alkyl, C₁-C₆-Perfluoralkyl, C₁-C₆-Alkoxy, C₇-C₁₂-Aralkyl, Halogen, SiR^{5a}R^{6a}R^{7a}, gegebenenfalls substituiertem C₆-C₁₀-Aryl, NR^{8a}R^{9a}, SR^{10a}, NO₂ tragen können, bedeuten,
R¹, R², R³, R⁴ unabhängig voneinander ausgewählt sind unter Wasserstoff, C₁-C₆-Alkyl, C₁-C₆-Perfluoralkyl, C₁-C₆-Alkoxy, C₇-C₁₂-Aralkyl, Halogen, SiR^{5b}R^{6b}R^{7b}, gegebenenfalls substituiertem C₆-C₁₀-Aryl, NR^{8b}R^{9b}, SR^{10b}, NO₂ und wobei
   R¹ oder R² und/oder R³ oder R⁴ gemeinsam mit A einen aromatischen oder nicht-aromatischen Cyclus bilden kann, bedeuten und
A für einen geradkettigen oder verzweigten und/oder cyclischen Kohlenwasserstoffrest mit 1 bis 25 C-Atomen steht, der gesättigt oder ein- oder mehrfach ungesättigt und/oder teilweise aromatisch sein kann und gegebenenfalls eines oder mehrere gleiche oder verschiedene Heteroatome, ausgewählt unter O, S, NR¹¹, und/oder eine oder mehrere gleiche oder verschiedene funktionelle Gruppen, ausgewählt unter den funktionellen Gruppen C(O), S(O), S(O)₂, aufweisen kann und gegebenenfalls einen oder mehrere gleiche oder verschiedene Substituenten, ausgewählt unter der Substituenten C₁-C₆-Alkyl, C₁-C₆-Perfluoralkyl, C₁-C₆-Alkoxy, C₁-C₁₀-Acyloxy, C₇-C₁₂-Aralkyl, Halogen, -SiR^{5c}R^{6c}R^{7c}, gegebenenfalls substituiertes C₆-C₁₀-Aryl, substituiertes oder unsubstituiertes C₂-C₁₀-Hetaryl, NR^{8c}R^{9c}, SR^{10c}, NO₂, C₁-C₁₂-Acyl, C₁-C₁₀-Carboxyl tragen kann, oder
   für einen C₆-C₁₅-Arylrest oder einen C₂-C₁₅-Heteroarylrest steht, der gegebenenfalls jeweils 1 bis 5 Substituenten, ausgewählt unter C₁-C₆-Alkyl, C₁-C₆-Perfluoralkyl, C₁-C₆-Alkoxy, C₇-C₁₂-Aralkyl, Halogen, SiR^{5d}R^{6d}R^{7d}, substituiertes oder unsubstituiertes C₆-C₁₀-Aryl, NR^{8d}R^{9d}, SR^{10d}, NO₂ tragen können, oder
   für eine funktionelle Gruppe oder ein Heteroatom ausgewählt aus der Gruppe -O-, -S-, -N(R¹¹)-, -S(O)-, -C(O)-, -S(O)₂-, -P(R¹¹)-, -(R¹¹)P(O)-und -Si(R¹²R¹³) steht,
wobei die Reste R^{5a}, R^{6a}, R^{7a}, R^{8a}, R^{9a}, R^{10a} bis R^{5d}, R^{6d}, R^{7d}, R^{8d}, R^{9d}, R^{10d}, R¹¹, R¹² und R¹³ jeweils unabhängig voneinander ausgewählt sind unter C₁-C₆-Alkyl, C₇-C₁₂-Aralkyl und/oder substituiertes oder unsubstituiertes C₆-C₁₀-Aryl und wobei die Reste R^{8a} und R^{9a}, R^{8b} und R^{9b}, R^{8c} und R^{9c}, R^{8d} und R^{9d} unabhängig voneinander jeweils gemeinsam auch einen cyclischen Kohlenwasserstoffrest mit 2 bis 8 Kohlenstoffatomen bilden können, der eines oder mehrere gleiche oder verschiedene Heteroatome, ausgewählt aus der Gruppe O, S, NR^{11a}, aufweisen kann und R^{11a} die für R¹¹ angegebenen Bedeutungen haben kann,
in freier und/oder komplexgebundener Form,
bei dem man
a) das Aluminium-haltige Reaktionsprodukt einer destillativen Auftrennung unter Erhalt eines an Isopulegol angereicherten Kopfprodukts und eines an Isopulegol abgereicherten Sumpfprodukts unterzieht,
b) das an Isopulegol abgereicherte Sumpfprodukt mit einer wässrigen Base unter Erhalt einer Aluminium-haltigen wässrigen Phase und einer die Hauptmenge der Liganden der Formel (I) enthaltenden organischen Phase in innigen Kontakt bringt,
c) den Liganden der Formel (I) aus der organischen Phase abtrennt.

In einer bevorzugten Ausführungsform des erfindungsgemäßen Verfahrens erfolgt das Abtrennen des Liganden der Formel (I) aus der organischen Phase durch Kristallisation.

Die nach dem erfindungsgemäßen Verfahren erhaltene Bis(diarylphenol)-Liganden der Formel (I) können üblicherweise ohne weitere Aufreinigungsschritte im Rahmen einer neuen Charge mit den entsprechenden Aluminiumverbindungen der Formeln (II) bzw. (III), wie im Folgenden definiert, zum reaktiven Katalysatorkomplex umgesetzt werden, wobei bei derartig wiederhergestellten Katalysatorkomplexen keine bzw. keine nennenswerte Abschwächung der Reaktivität festzustellen ist.

Die Bis(diarylphenol)-Liganden der Formel (I) weisen zwei Phenolsysteme auf, die jeweils in beiden ortho-Positionen zur phenolischen Hydroxy-Gruppe durch Aromaten bzw. Heteroaromaten (Ar¹ bis Ar⁴) substituiert sind und über ein Strukturelement A miteinander verknüpft sind und gegebenenfalls noch weitere Substituenten (R¹ bis R⁴) tragen können.

Die aromatischen bzw. heteroaromatischen Substituenten Ar¹ bis Ar⁴ können unabhängig voneinander gleich oder verschieden sein. Bevorzugt sind die beiden jeweils an ein Phenolsystem gebundenen Substituenten (Ar¹ und Ar² bzw. Ar³ und Ar⁴) paarweise gleich. Insbesondere bevorzugt sind alle vier Substituenten Ar¹ bis Ar⁴ gleich.

Die genannten Substituenten Ar¹ bis Ar⁴ sind Arylreste mit 6 bis 15, bevorzugt 6 bis 10 Kohlenstoffatomen oder Heteroarylreste mit 2 bis 15, bevorzugt 3 bis 10 Kohlenstoffatomen, im aromatischen Ringsystem. Arylreste mit 6 bis 15 Kohlenstoffatomen sind beispielsweise Phenyl, Naphthyl, Anthracenyl, bevorzugt Phenyl und Naphthyl.

Die genannten Heteroarylreste mit 2 bis 15 Kohlenstoffatomen weisen 1 bis etwa 6, in der Regel 1 bis 3, gleiche oder verschiedene Heteroatome, die ausgewählt sind aus der Gruppe der Heteroatome O, S und N, auf. Beispielhaft seien dafür die folgenden Heteroarylreste genannt: 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl, 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl, Benzofuryl, Isobenzofuryl, Benzothienyl, Indolyl, Isoindolyl, Carbazolyl, Pyridyl, Chinolyl, Isochinolyl und Pyrazyl. Bevorzugte Heteroarylreste sind beispielsweise: 2-Furyl, 2-Pyridyl, 2-Imidazoyl.

Die vorstehend für Ar¹ bis Ar⁴ genannten Aryl bzw. Heteroarylreste können jeweils unabhängig voneinander unsubstituiert sein oder 1 bis etwa 7, bevorzugt 1 bis 3, insbesondere 1 oder 2, gleiche oder verschiedene Substituenten tragen, die ausgewählt sind aus der Gruppe der Substituenten: C₁-C₆-Alkyl, C₁-C₆-Perfluoralkyl, C₁-C₆-Alkoxy, C₇-C₁₂-Aralkyl, Halogen, -SiR^{5a}R^{6a}R^{7a}, substituiertes oder unsubstituiertes C₆-C₁₀-Aryl, -NR^{8a}R^{9a}, -SR^{10a}, -NO₂, wobei die Reste R^{5a}, R^{6a}, R^{7a}, R^{8a}, R^{9a}, R^{10a} und R¹¹ bis R¹³ jeweils unabhängig voneinander für C₁-C₆-Alkyl, C₇-C₁₂-Aralkyl und/oder substituiertes oder unsubstituiertes C₆-C₁₀-Aryl stehen, und die Reste R^{8a} und R^{9a} unabhängig voneinander jeweils gemeinsam auch einen cyclischen Kohlenwasserstoffrest mit 2 bis 8 Kohlenstoffatomen bilden können, der eines oder mehrere gleiche oder verschiedene Heteroatome, ausgewählt aus der Gruppe O, S und NR^{11a}, aufweisen kann und R^{11a} die für R¹¹ angegebenen Bedeutungen haben kann.
Dabei können den genannten Substituenten im Rahmen der gesamten vorliegenden Erfindung die nachfolgend beispielhaft genannten Bedeutungen zukommen:
C₁-C₆-Alkyl wie beispielsweise Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;

C₁-C₆-Perfluoralkyl wie beispielsweise Trifluormethyl, Pentafluorethyl, Heptafluorpropyl, Heptafluorisopropyl, Nonafluorbutyl;
C₁-C₆-Alkoxy wie beispielsweise Methoxy, Ethoxy, Propoxy, 1-Methylethoxy, Butoxy, 1-Methylpropoxy, 2-Methylpropoxy und 1,1-Dimethylethoxy, Pentoxy, 1-Methylbutoxy, 2-Methylbutoxy, 3-Methoxylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
C₇-C₁₂-Aralkyl wie beispielsweise Benzyl, 1-Phenylethyl, 2-Phenylethyl;
C₁-C₁₀-Acyloxy wie beispielsweise Acetyloxy, Propionyloxy;

C₁-C₁₀-Carboxyl wie beispielsweise Methoxycarbonyl, Ethoxycarbonyl, Propyloxycarbonyl, Isopropyloxycarbonyl;

C₁-C₁₀-Acyl wie beispielsweise Formyl, Acetyl, Propionyl.

Der Ausdruck "substituiertes oder unsubstituiertes C₆-C₁₀-Aryl" steht im Rahmen der vorliegenden Erfindung für Arylreste, die wie vorstehend genannt einen oder mehrere, in der Regel 1 bis etwa 3, gleiche oder verschiedene Substituenten aufweisen, wobei die Substituenten beispielsweise unter C₁-C₆-Alkyl, C₁-C₆-Perfluoralkyl, C₁-C₆-Alkoxy, C₇-C₁₂-Aralkyl, Halogen, Silyl, Dialkylamino und Nitro ausgewählt sein können.

Der Begriff "Halogen" steht im Rahmen der vorliegenden Erfindung für Fluor, Chlor, Brom und Jod, bevorzugt für Fluor und Chlor.

Unter den Substituenten -SiR^{5a}R^{6a}R^{7a} bis -SiR^{5d}R^{6d}R^{7d} sind im Rahmen der vorliegenden Erfindung jeweils Silylsubstituenten mit jeweils unabhängig voneinander drei gleichen oder verschiedenen Resten, die ausgewählt sind unter den Resten C₁-C₆-Alkyl, C₇-C₁₂-Aralkyl und substituiertes oder unsubstituiertes C₆-C₁₀-Aryl zu verstehen. Beispielhaft seien dafür etwa die Silylsubstituenten Trimethylsilyl, Triethylsilyl, tert-Butyldimethylsilyl und tert-Butyl-diphenylsilyl genannt.

Die Substituenten -NR^{8a}R^{9a} bis -NR^{8d}R^{9d} stehen im Rahmen der vorliegenden Erfindung jeweils für Amino-Substituenten, die jeweils unabhängig voneinander zwei gleiche oder verschiedene, bevorzugt zwei gleiche Rest tragen, die ausgewählt sind unter den vorstehend beschriebenen Resten C₁-C₆-Alkyl, C₇-C₁₂-Aralkyl und/oder substituiertes oder unsubstituiertes C₆-C₁₀-Aryl. Beispielhaft seien als Amino-Substituenten genannt: Dimethylamino, Diethylamino, Dibenzylamino, Diallylamino, Diisopropylamino. Die Reste R^{8a} und R^{9a} bis R^{8d} und R^{9d} können im Rahmen der vorliegenden Erfindung unabhängig voneinander jeweils gemeinsam auch einen cyclischen Kohlenwasserstoffrest mit 2 bis 8 Kohlenstoffatomen bilden, der eines oder mehrere gleiche oder verschieden Heteroatome, ausgewählt aus der Gruppe O, S, NR^{11a}, aufweisen kann. Der Rest R^{11a} kann dabei wie vorstehend beschriebenes C₁-C₆-Alkyl, C₇-C₁₂-Aralkyl und/oder substituiertes oder unsubstituiertes C₆-C₁₀-Aryl bedeuten. Beispielhaft seien für diese cyclischen Substituenten R^{8a} und R^{9a} bis R^{8d} und R^{9d} etwa genannt: Piperidinyl, Morpholinyl, N-Methylpiperazinyl, N-Benzylpiperazinyl.

Bei den Substituenten -SR^{10a} steht der Rest R^{10a} für wie vorstehend definiertes C₁-C₆-Alkyl, C₇-C₁₂-Aralkyl und/oder substituiertes oder unsubstituiertes C₆-C₁₀-Aryl, bevorzugt für Methyl, Ethyl, Isopropyl, Phenyl, Benzyl.

Im Rahmen der vorliegenden Erfindung sind bevorzugte aromatische oder heteroaromatische Substituenten Ar¹, Ar², Ar³, Ar⁴ beispielsweise Phenyl, 4-Methylphenyl, 2,4,6-Trimethylphenyl, Naphthyl, 2-Fluorphenyl, 4-Fluorphenyl, 4-Chlorphenyl, 4-Bromphenyl, 3-Fluorphenyl, 3-Chlorphenyl, 3,5-Difluorphenyl, 3,5-Dichlorphenyl, 2,3,6-Trichlorphenyl, 2,4,6-Trichlorphenyl, 2-Methylphenyl, 4-Methylphenyl, 2,4,5-Trimethylphenyl, 2,4,6-Trimethylphenyl. 2-Isopropylphenyl, 4-Isopropylphenyl, 4-tert.-Butylphenyl, 4-n-Butylphenyl, 3-Trifluormethylphenyl, 4-Trifluormethylphenyl, 3,5-Bis(trifluormethyl)phenyl, 4-Arylphenyl, 3-Nitrophenyl, bevorzugt 4-Fluorphenyl, 4-Chlorphenyl, 3-Chlorphenyl, 3,5-Dichlorphenyl, 3-Trifluormethylphenyl, 4-Trifluormethylphenyl. Im Rahmen einer bevorzugten Ausführungsform sind die Reste Ar¹, Ar², Ar³, Ar⁴ gleich und bedeuten bevorzugt 4-Fluorphenyl, 4-Chlorphenyl, 3-Chlorphenyl, 3,5-Dichlorphenyl, 3-Trifluormethylphenyl, 4-Trifluormethylphenyl, insbesondere bevorzugt Phenyl.

Die zu den jeweiligen phenolischen Hydroxygruppen meta- oder para-ständigen Substituenten R¹, R², R³, R⁴ können erfindungsgemäß gleich oder verschieden, bevorzugt gleich, sein und jeweils unabhängig voneinander Wasserstoff und/oder wie vorstehend genanntes C₁-C₆-Alkyl, C₁-C₆-Perfluoralkyl, C₁-C₆-Alkoxy, C₇-C₁₂-Aralkyl, Halogen, -SiR^{5b}R^{6b}, R^{7b}, substituiertes oder unsubstituiertes C₆-C₁₀-Aryl, -NR^{8b}R^{9b}, -SR^{10b} und/oder -NO₂ bedeuten.

Als bevorzugte Reste R¹, R², R³, R⁴ seien genannt: Methyl, Ethyl, Isopropyl, Halogen, insbesondere Fluor und/oder Chlor, Trifluormethyl, Phenyl, Methoxy, Nitro. Bevorzugt sind die Reste R¹, R², R³, R⁴ gleich und bedeuten insbesondere bevorzugt Wasserstoff.

Die Reste R¹ oder R² und/oder R³ oder R⁴ können gemeinsam mit dem Strukturelement A auch einen cyclischen aromatischen oder nicht-aromatischen Cyclus bilden. In diesen Fällen weisen die erfindungsgemäß einzusetzenden Bis(diarylphenol)-Liganden der Formel (I) ein tricyclisches Grundgerüst, beispielweise ein Anthracen-Grundgerüst der Formel (X) oder Grundgerüste des Typs (XI), auf:

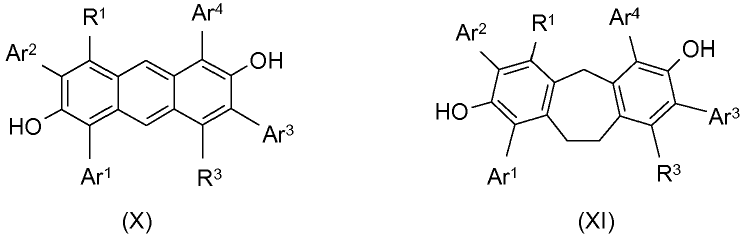

Weitere strukturelle Abwandlungen dieser tricyclischen Grundgerüste, gegebenenfalls auch solchen, die Heteroatome im Grundgerüst aufweisen, erschließen sich dem Fachmann und gehören zur Gruppe der erfindungsgemäß einsetzbaren Bis(diarylphenol)-Liganden.

Das Strukturelement A in Formel (I) kann für einen geradkettigen oder verzweigten und/oder cyclischen Kohlenwasserstoffrest mit 1 bis 25 Kohlenstoffatomen stehen, der gesättigt oder ein- oder mehrfach ungesättigt, normalerweise 1- bis etwa 6-fach ungesättigt sein kann und/oder teilweise aromatisch sein kann. Die genannten Kohlenwasserstoffreste können gegebenenfalls eines oder mehrere, in der Regel 1 bis 3, gleiche oder verschiedene Heteroatome, ausgewählt aus der Gruppe der Heteroatome O, S und NR¹¹ und/oder eine oder mehrere gleiche oder verschiedene funktionelle Gruppen, ausgewählt aus der Gruppe der funktionellen Gruppen C(O), S(O) und S(O)₂, aufweisen und gegebenenfalls einen oder mehrere gleiche oder verschiedene Substituenten, ausgewählt aus der Gruppe der Substituenten C₁-C₆-Alkyl, C₁-C₆-Perfluoralkyl, C₁-C₆-Alkoxy, C₁-C₁₀-Acyloxy, C₇-C₁₂-Aralkyl, Halogen, -SiR^{5c}R^{6c}R^{7c}, substituiertes oder unsubstituiertes C₆-C₁₀-Aryl, substituiertes oder unsubstituiertes C₂-C₁₀-Hetaryl, -NR^{8c}R^{9c}, -SR^{10c}, -NO₂, C₁-C₁₂-Acyl und C₁-C₁₀-Carboxyl tragen.

Bevorzugt steht das Strukturelement A in Formel (I) für einen geradkettigen oder verzweigten und/oder cyclischen Kohlenwasserstoffrest mit 1 bis 25, bevorzugt 1 bis 15, und besonders bevorzugt 1 bis 10 Kohlenstoffatomen , der gesättigt oder ein- bis dreifach ungesättigt sein kann und/oder teilweise aromatisch sein kann. Die bevorzugten Kohlenwasserstoffreste können gegebenenfalls eines oder mehrere, in der Regel 1 bis 3, gleiche oder verschiedene Heteroatome, ausgewählt aus der Gruppe der Heteroatome O, S und NR¹¹ und/oder eine oder mehrere C(O)-Gruppen aufweisen und gegebenenfalls einen oder mehrere gleiche oder verschiedene Substituenten, ausgewählt aus der Gruppe der Substituenten C₁-C₆-Alkyl, C₁-C₆-Perfluoralkyl, C₁-C₆-Alkoxy, C₁-C₁₀-Acyloxy, C₇-C₁₂-Aralkyl, Halogen, substituiertes oder unsubstituiertes C₆-C₁₀-Aryl, -NO₂, C₁-C₁₂-Acyl und C₁-C₁₀-Carboxyl tragen.

Als Beispiele für Strukturelemente A in der Formel (I) seien ohne jeden einschränkenden Charakter die folgenden Strukturelemente 1 bis 44 genannt, wobei die Schlangenlinien jeweils, wie im Rahmen der gesamten vorliegenden Offenbarung, die Anknüpfungsstellen zum Rest der jeweiligen Ligandenstruktur markieren:

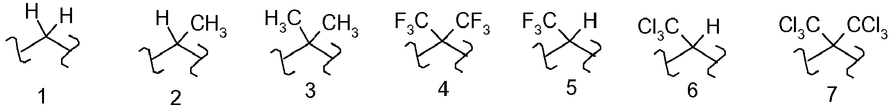

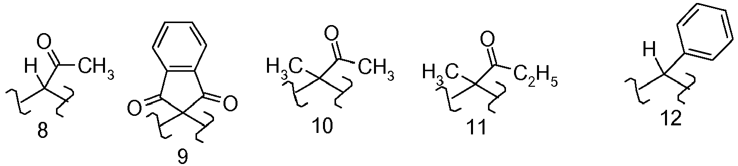

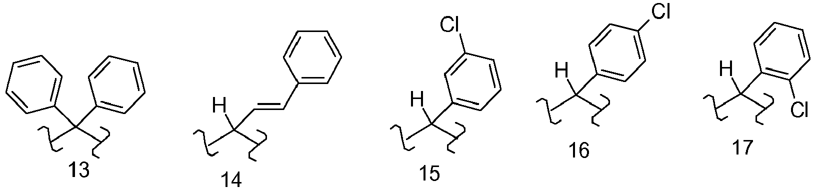

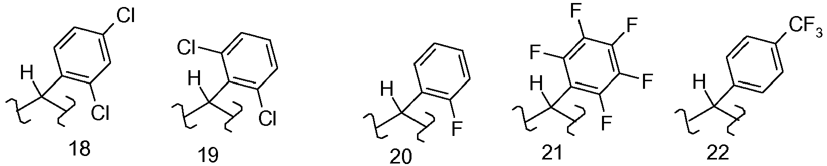

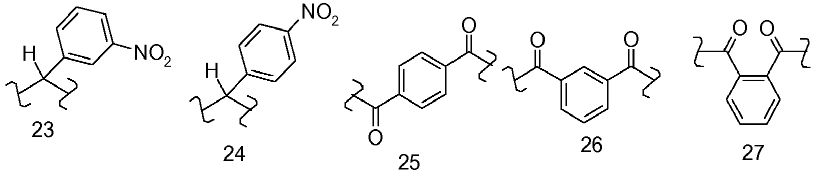

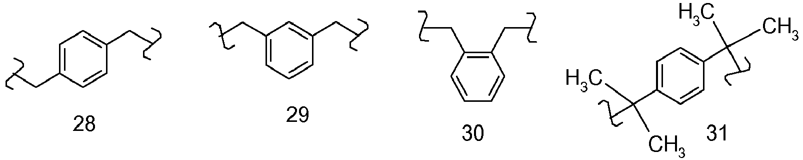

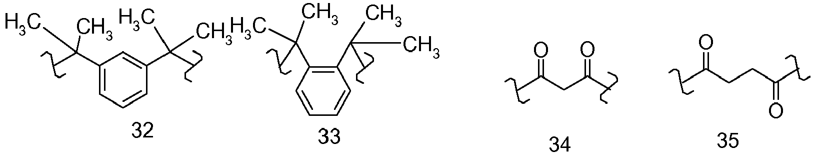

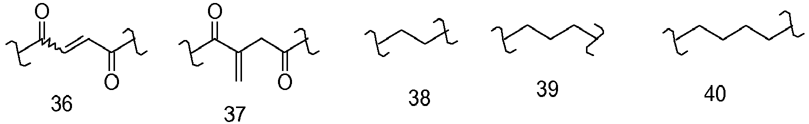

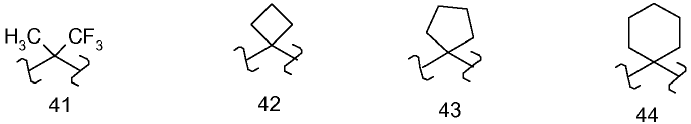

Auch die dargestellten Strukturelemente 1 bis 44 können jeweils die wie vorstehend bezeichneten Substituenten tragen und gegebenenfalls weitere, üblicherweise 1 oder 2 ethylenische Doppelbindungen aufweisen.

Das Strukturelement A kann auch für einen Arylrest mit 6 bis 15, bevorzugt 6 bis 10 Kohlenstoffatomen, speziell einen Phenylen-, Naphthylen- oder Anthracenylenrest stehen oder für einen wie vorstehend definierten Heteroarylrest mit 2 bis 15, bevorzugt 3 bis 10 Kohlenstoffatomen stehen.

Die genannten Aryl- bzw. Heteroarylreste können gegebenenfalls jeweils 1 bis 5 Substituenten tragen, die ausgewählt sind aus der Gruppe der wie vorstehend beschriebenen Substituenten C₁-C₆-Alkyl, C₁-C₆-Perfluoralkyl, C₁-C₆-Alkoxy, C₇-C₁₂-Aralkyl, Halogen, -SiR^{5d}R^{6d}, R^{7d}, substituiertes oder unsubstituiertes C₆-C₁₀-Aryl, -NR^{8d}R^{9d}, SR^{10d} und NO₂.

Des Weiteren kann das Strukturelement A auch für eine funktionelle Gruppe oder ein Heteroatom stehen, die ausgewählt sind aus der Gruppe -O-, -S-, -N(R¹¹)-, -S(O)-, -C(O)-, -S(O)₂-, -P(R¹¹)-, -(R¹¹)P(O)-, -OP(O)O-, -OP(O)₂O- und -Si(R¹²)(R¹³)-, wobei die Reste R¹¹, R¹², R¹³ unabhängig voneinander jeweils für wie vorstehend beschriebenes C₁-C₆-Alkyl, C₇-C₁₂-Aralkyl und/oder substituiertes oder unsubstituiertes C₆-C₁₀-Aryl stehen. Im Rahmen dieser Gruppe steht das Strukturelement A bevorzugt für -O-, -S-, -S(O)-, -S(O)₂- oder -Si(R¹²)(R¹³)-.

Im Rahmen der vorliegenden Erfindung umfasst der Begriff "Ligand in freier oder komplexgebundener Form" sowohl die freie Form des Liganden als auch sämtliche denkbaren Formen, die unter den Verfahrensbedingungen in die freie Form überführbar sind. Beispielhaft hierfür seinen Alkoholate des Liganden genannt, die durch die basische Hydrolyse in die freie Form des Liganden überführt werden.

Im Rahmen der vorliegenden Erfindung umfasst der Ausdruck "wässrige Base" allgemein wässrige Lösungen, deren pH-Wert größer als 7 ist. Insbesondere handelt es sich um wässrige Lösungen von Alkali- und Erdalkalimetallhydroxiden, speziell wässrige Lösungen von KOH und NaOH.

Der Ausdruck "Aluminium-haltiges Reaktionsprodukt" beschreibt im Rahmen der vorliegenden Erfindung ein Reaktionsprodukt, das wenigstens eine Verbindung umfasst, die Aluminium ionisch, kovalent oder komplexgebunden enthält. Dabei handelt es sich um Verbindungen des Aluminiums, wie sie unter den Bedingungen des erfindungsgemäßen Verfahrens aus den bei der Cyclisierung von Citronellal eingesetzten Verbindungen der Formel (R¹⁴)₃₋ₚAlHₚ (II) oder MAlH₄ (III), wie hierunter definiert, resultieren.

Unter dem Ausdruck "Hauptmenge" soll im Rahmen der vorliegenden Erfindung ein prozentualer Mengenanteil an der vorhandenen Gesamtmenge einer Verbindung verstanden werden, der größer 50 %, bevorzugt größer 80 % und insbesondere bevorzugt größer 90 % ist.

### Schritt a):

In Schritt a) des erfindungsgemäßen Verfahrens wird das Aluminium-haltige Reaktionsprodukt aus der Herstellung von Isopulegol durch Cyclisierung von Citronellal einer destillativen Auftrennung unter Erhalt eines an Isopulegol angereicherten Kopfprodukts und eines an Isopulegol abgereicherten Sumpfprodukts unterzogen.

In einer speziellen Ausführungsform wird in Schritt a) ein höher als das Isopulegol siedendes Lösungsmittel eingesetzt. Somit kann eine unerwünschte thermische Beanspruchung des Sumpfinhalts vermieden werden. Insbesondere liegen die darin enthaltenen Liganden der Formel (I) während der Abtrennung des Isopulegols nicht frei von Lösungsmittel vor. Das höhersiedende Lösungsmittel kann dem Aluminium-haltigen Reaktionsprodukt vor und/oder während der destillativen Auftrennung hinzugefügt werden. Vorzugsweise wird ein höhersiedendes Lösungsmittel eingesetzt, dessen Siedepunkt unter den Bedingungen der Destillation über dem Siedepunkt des Isopulegols liegt. Bevorzugt liegt der Siedepunkt des zugeführten Lösungsmittels unter den Bedingungen der Destillation wenigstens 5 °C, bevorzugt wenigstens 10 °C und insbesondere wenigstens 20 °C, über dem Siedepunkt des Isopulegols.

Bevorzugte höhersiedende Lösungsmittel, die einen solchen Siedepunkt aufweisen, sind beispielsweise Kohlenwasserstoffe, wie Phenylcyclohexan, Benzyltoluol, Dibenzyltoluol, 1-Methylnaphthalin und Tridecan, 1-Decanol, 1,2-Propylencarbonat, Ether, wie Diethylenglycoldibutylether, Tetraethylenglycoldimethylether und Dibenzylether, sowie technische Gemische dieser Lösungsmittel. Insbesondere bevorzugt sind Gemische, die als Hauptbestandteil Phenylcyclohexan enthalten.

Bei Einsatz wenigstens eines höhersiedenden Lösungsmittels wird als an Isopulegol abgereichertes Sumpfprodukt in Schritt a) eine organische Phase erhalten, umfassend das höhersiedende Lösungsmittel, die Hauptmenge der Liganden der Formel (I) sowie gegebenenfalls wenigstens eine Aluminium-haltige Verbindung.

Vorzugsweise erfolgt die destillative Abtrennung von Isopulegol in Schritt a) bei einer Sumpftemperatur von vorzugsweise höchstens 250 °C, bevorzugt höchstens 150 °C und besonders bevorzugt höchstens 100 °C. Die untere Sumpftemperatur ist in der Regel unkritisch und beträgt im Allgemeinen wenigstens 0 °C, vorzugsweise wenigstens 20 °C. Zur Einhaltung dieser Maximaltemperaturen kann die Destillation gewünschtenfalls unter einem geeigneten Vakuum durchgeführt werden.

Der Druck in Schritt a) der erfindungsgemäßen Verfahren liegt unabhängig von der speziellen Ausführungsform im Allgemeinen in einem Bereich von 0,1 bis 1500 mbar, bevorzugt in einem Bereich von 1 bis 500 mbar und besonderst bevorzugt in einem Bereich von 5 bis 100 mbar.

Unabhängig von der Zusammensetzung des Aluminium-haltigen Reaktionsprodukts aus der Cyclisierung von Citronellal und vom Einsatz eines höhersiedenden Lösungsmittels kann die destillative Abtrennung des Isopulegols kontinuierlich oder diskontinuierlich, vorzugsweise kontinuierlich erfolgen. In einer geeigneten Vorgehensweise wird dem Reaktionsprodukt aus der Cyclisierung von Citronellal das höhersiedende Lösungsmittel vor der destillativen Auftrennung zugegeben und im Verlauf der Destillation die im Sumpf vorhandene Menge an hochsiedendem Lösungsmittel im Weiteren konstant gehalten.

Zur destillativen Auftrennung in Schritt a) können die üblichen dem Fachmann bekannten Vorrichtungen eingesetzt werden (siehe z. B. Sattler, Thermische Trennverfahren, 2. Auflage 1995, Weinheim, S. 135ff; Perry's Chemical Engineers Handbook, 7. Auflage 1997, New York, Section 13). Dazu zählen Destillationssäulen und -kolonnen, die mit Packungen, Einbauten etc. versehen sein können. Die eingesetzten Destillationssäulen können trennwirksame Einbauten enthalten, wie Trennböden, z. B. Lochböden, Glockenböden oder Ventilböden, geordnete Packungen, z. B. Blech- oder Gewebepackungen, oder regellose Schüttungen von Füllkörpern. Die in der/den eingesetzten Kolonne(n) notwendige Stufenzahl und das Rücklaufverhältnis richten sich im Wesentlichen nach den Reinheitsanforderungen und der relativen Siedelage der Bestandteile des Aluminium-haltigen Reaktionsprodukts aus der Herstellung von Isopulegol durch Cyclisierung von Citronellal und des höhersiedenden Lösungsmittels, wobei der Fachmann die konkreten Auslegungs- und Betriebsdaten nach bekannten Methoden ermitteln kann. Die destillative Auftrennung kann z. B. in einer oder mehreren miteinander gekoppelten Destillationskolonnen erfolgen.

Zur destillativen Auftrennung in Schritt a) eignen sich ebenfalls übliche Verdampfer, vorzugsweise Verdampfer mit Zwangsumlauf, besonders bevorzugt Fallfilmverdampfer.

In Abhängigkeit gegebenenfalls enthaltener zusätzlicher Komponenten im Aluminium-haltigen Reaktionsprodukt aus der Cyclisierung von Citronellal kann die Zusammensetzung des bei der destillativen Auftrennung erhaltenen Kopfprodukts es erforderlich machen, dieses gegebenenfalls einem weiteren Aufarbeitungsschritt zu unterziehen.

In einer speziellen Ausführungsform des erfindungsgemäßen Verfahrens zur Aufbereitung eines Aluminium-haltigen Reaktionsprodukts aus der Herstellung von Isopulegol durch Cyclisierung von Citronellal, enthält das Reaktionsprodukt zusätzlich ein niedriger siedendes Lösungsmittel (iii).

Der Ausdruck "niedriger siedendes Lösungsmittel (iii)" bezieht sich im Rahmen der vorliegenden Erfindung auf den Siedepunkt des Isopulegols. Insbesondere eignen sich hierfür solche Lösungsmittel oder Lösungsmittelgemische, die unter den Bedingungen der destillativen Auftrennung einen Siedepunkt aufweisen, der wenigstens 5 °C, bevorzugt 10 °C und insbesondere 20 °C, unter dem des Isopulegols bei den jeweiligen Bedingungen liegt.

Bevorzugte Lösemittel mit einem solchen Siedepunkt sind im Rahmen der vorliegenden Erfindung inerte organische Lösungsmittel oder Mischungen davon, wie beispielsweise aromatische Lösungsmittel, z. B. Toluol, Ethylbenzol oder Xylol, halogenierte Lösungsmittel, z. B. Dichlormethan , Dichlorethan oder Chlorbenzol, aliphatische Lösungsmittel, z. B. Pentan, Hexan oder Cyclohexan, Ether, z. B. Tetrahydrofuran, Diethylether, Methyl-tert-Butylether, Ester, z. B. Essigsäureethylester, oder Dimethylformamid (DMF), Dimethylsulfoxid (DMSO) und dergleichen mehr. Besonderst bevorzugt handelt es sich um Toluol.

Enthält das aufzuarbeitende Aluminium-haltige Reaktionsprodukt ein solches niedriger siedendes Lösungsmittel, so wird dieses in einer geeigneten Ausführungsform vor der destillativen Abtrennung des Isopulegols zumindest teilweise aus dem Reaktionsprodukt entfernt. Die Abtrennung des niedriger siedenden Lösungsmittels erfolgt vorzugsweise ebenfalls destillativ. In Abhängigkeit vom Siedepunkt des niedriger siedenden Lösungsmittels können die üblichen zuvor genannten Destillationsvorrichtungen eingesetzt werden.

In einer weiteren geeigneten Ausführungsform erfolgt die destillative Auftrennung des Aluminium-haltigen Reaktionsprodukts in Schritt a) unter Erhalt eines an Isopulegol angereicherten Kopfprodukts, das gleichzeitig zumindest einen Teil, vorzugsweise die Hauptmenge des niedriger siedenden Lösungsmittels, enthält. In diesem Fall kann das Kopfprodukt einer weiteren Auftrennung, bevorzugt ebenfalls destillativ, unterzogen werden.

Das abgetrennte niedriger siedende Lösungsmittel wird mit Vorteil in die Cyclisierung des Citronellals zurückgeführt, in dem es als Lösungsmittel eingesetzt wird. Auf diese Weise erfordert das erfindungsgemäße Verfahren - bis auf Ergänzungen, die durch unvermeidliche Verluste erforderlich werden - die lediglich einmalige Bereitstellung einer Menge des niedriger siedenden Lösungsmittels.

In einer speziellen Ausführungsform des erfindungsgemäßen Verfahrens zur Aufbereitung eines Aluminium-haltigen Reaktionsprodukts aus der Herstellung von Isopulegol durch Cyclisierung von Citronellal, enthält das Reaktionsprodukt zusätzlich einen Hilfsstoff (iv).

Der Begriff "Hilfsstoff (iv)" bezieht sich im Rahmen der vorliegenden Erfindung auf Verbindungen, die bei der Cyclisierung von Citronellal zugesetzt werden, um unerwünschte Nebenreaktionen zu unterdrücken. Bevorzugt sind die Hilfsstoffe (iv) ausgewählt unter organischen Säuren, Carbonsäureanhydriden, Aldehyden, Ketonen und Vinylethern.

Speziell sind die Hilfsstoffe (iv) ausgewählt unter Säuren, vorzugsweise organische Säuren. Beispielhaft seien als organische Säuren genannt: Essigsäure, Propionsäure, Benzoesäure, Toluolsulfonsäure, Methansulfonsäure, bevorzugt Essigsäure.

In einer weiteren speziellen Ausführungsform der vorliegenden Erfindung sind die Hilfsstoffe (iv) ausgewählt unter Carbonsäureanhydriden, Aldehyden, Ketonen und Vinylethern.

Die Hilfsstoffe (iv) der genannten Substanzklassen können jeweils einzeln oder in Form von Gemischen in dem aufzuarbeitenden Reaktionsprodukt vorliegen. Bevorzugte Gemischen sind solche, die aus Verbindungen einer Substanzklasse bestehen. Besonders bevorzugt enthält das Reaktionsprodukt einen einzelnen Hilfsstoff.

Vorzugsweise werden die im Reaktionsprodukt aus der Cyclisierung von Citronellal enthaltenen Hilfsstoffe (iv) ebenfalls zumindest teilweise entfernt und soweit wie möglich in die Cyclisierung von Citronellal zurückgeführt.

Weisen die Hilfsstoffe (iv) unter den Bedingungen der Destillation einen Siedepunkt auf, der unterhalb oder nur geringfügig, d. h. weniger als 30 °C, über dem Siedepunkt des Isopulegols liegt, können diese durch Destillation aus der ausreagierten Mischung weitgehend und in dem Maße, in dem sie nicht gegebenenfalls selbst umgesetzt wurde, zurück gewonnen werden. In Abhängigkeit vom Siedepunkt des Hilfsstoffs können die üblichen zuvor genannten Destillationsvorrichtungen eingesetzt werden.

Weisen die Hilfsstoffe (iv) unter den Bedingungen der Destillation einen Siedepunkt auf, der deutlich oberhalb, d. h. wenigstens 30 °C, über dem Siedepunkt des Isopulegols liegt, verbleiben diese im Sumpfprodukt und werden gegebenenfalls in Schritt b) des erfindungsgemäßen Verfahrens entfernt, wenn Ihre physikalischen Eigenschaften dies erlauben.

In einer weiteren geeigneten Ausführungsform erfolgt die destillative Auftrennung des Reaktionsprodukts in Schritt a) unter Erhalt eines an Isopulegol angereicherten Kopfprodukts, das gleichzeitig zumindest einen Teil, vorzugsweise die Hauptmenge des Hilfsstoffs (iv), enthält. Gegebenenfalls kann dieses Hauptprodukt ein niedriger siedendes Lösungsmittel, wie zuvor ausgeführt, enthalten. In diesem Fall kann das Kopfprodukt einer weiteren Auftrennung, bevorzugt ebenfalls destillativ, unterzogen werden. Der abgetrennte Hilfsstoff (iv) wird, gegebenenfalls gemeinsam mit dem niedriger siedenden Lösungsmittel, mit Vorteil in die Cyclisierung des Citronellals zurückgeführt, in der er z. B. zur Unterdrückung unerwünschter Nebenreaktionen eingesetzt wird. Auf diese Weise erfordert das erfindungsgemäße Verfahren - bis auf Ergänzungen, die durch unvermeidliche Verluste erforderlich werden - die lediglich einmalige Bereitstellung einer Menge des Hilfsstoffs (iv).

Die Isopulegolabtrennung, die Zufuhr des höhersiedenden Lösungsmittels und gegebenenfalls die Leichtsiederabtrennung, d. h. die Abtrennung von gegebenenfalls vorhandenen Lösungsmitteln und flüchtigen Hilfsstoffen aus der Cyclisierung von Citronellal, können auf unterschiedliche Weisen kombiniert werden:

In einer geeigneten Ausführungsform verwendet man zur Destillation eine so genannte Trennwandkolonne, d. h. Zulaufstelle und ein Seitenabzug befinden sich auf entgegen gesetzten Seiten einer Trennwand, die sich über einen Abschnitt der Längsausdehnung der Kolonne erstreckt. Derartige Destillationskolonnen, die eine Trennwand enthalten, sind dem Fachmann an sich bekannt. Sofern sich Seitenabzug und Zulauf im Bereich der Trennwand befinden, entsteht eine zu einer Brugma- oder Petlyuk-Schaltung analoge Schaltung. Derartige Destillationen unter Verwendung von Trennwandkolonnen sind in der DE-A-33 02 525 und EP-A-0 804 951 beschrieben.

In diesem Fall kann z. B. als Kopfprodukt eine an Leichtsiedern angereicherte Fraktion und als Seitenabzug einen den Hauptteil an Isopulegol enthaltenden Strom abgezogen werden. Das höhersiedende Lösungsmittel wird unterhalb der Zulaufstelle, bevorzugt in den Sumpf der Kolonne oder kurz oberhalb des Sumpfes, zugefahren. Eine Lösung der Hauptmenge des Liganden der Formel (I) in dem höhersiedenden Lösungsmittel fällt als Sumpfprodukt an.

In einer alternativen Ausführungsform verwendet man zur Destillation gekoppelte Kolonnen. Diese Ausführungsform kann von Vorteil sein, wenn das Reaktionsprodukt der Cyclisierung von Citronellal ein Lösungsmittel und/oder einen flüchtigen Hilfsstoff enthält, wie im Folgenden näher ausgeführt.

In diesem Fall können Gemische aus Isopulegol und niedriger oder geringfügig höhersiedenden Lösungsmitteln und/oder Hilfsstoff (iv) das Kopfprodukt der ersten Kolonne bilden und in der zweiten Kolonne einer Auftrennung unter Erhalt eines zumindest die Hauptmenge des Isopulegols enthaltenden Stroms und eines an Isopulegol abgereicherten, die niedriger siedenden Lösungsmittel und/oder Hilfsstoffe der Cyclisierung enthaltenden Stroms, unterzogen werden.

Ströme, die niedriger siedende Lösungsmittel (iii) und Hilfsstoff (iv) der Cyclisierung enthalten, können in der Regel ohne weitere Auftrennung in die Cyclisierung zurückgeführt werden.

Die Liganden der Formel (I) fallen, gegebenenfalls in Form ihrer Komplexe oder anderer Derivate, als Sumpfprodukt der ersten Kolonne an.

### Schritt b):

In Schritt b) des erfindungsgemäßen Verfahrens wird das an Isopulegol abgereicherte Sumpfprodukt mit einer wässrigen Base unter Erhalt einer Aluminium-haltigen wässrigen Phase und einer die Hauptmenge der Liganden der Formel (I) enthaltenden organischen Phase in innigen Kontakt gebracht. Bevorzugte wässrige Basen sind die zuvor genannten.

Das in Schritt a) erhaltene, an Isopulegol abgereicherte Sumpfprodukt kann neben dem Liganden der Formel (I) in freier oder komplexgebundener Form wenigstens eine weitere schwerflüchtige Komponente enthalten. Dazu zählen z. B. in Schritt a) zugesetzte höhersiedende Lösungsmittel, die Umsetzungsprodukte der zur Cyclisierung von Citronellal zu Isopulegol eingesetzten Aluminium-haltigen Verbindungen sowie gegebenenfalls in Schritt a) nicht abgetrennte Hilfsstoffe (iv). Da sich Aluminium-haltige Komponenten und/oder die Hilfsstoffe (iv) insbesondere bei einem kontinuierlichen Verfahren anreichern und sich speziell auf die Ausbeute und Reinheit des Abtrennens in Schritt c) negativ auswirken, ist es vorteilhaft, diese Verbindungen möglichst vollständig zu entfernen. Dies gilt speziell für die Aluminium-haltigen Verbindungen.

Das Inkontaktbringen in Schritt b) erfolgt vorzugsweise durch Extraktion. Die Zahl der Extraktionsstufen liegt vorzugsweise in einem Bereich von 1 bis 20 Stufen.

Als Extraktionsmittel dienen die zuvor genannten wässrigen Basen. Daher werden diese Ausdrücke im Rahmen der vorliegenden Erfindung synonym verwendet.

Zur Extraktion bringt man das an Isopulegol abgereicherte Sumpfprodukt aus Schritt a) mit einer wässrigen Base innig in Kontakt. Nach Trennung der Phasen erhält man eine die Hauptmenge des Liganden der Formel (I) enthaltende Phase und eine an Aluminium-haltigen Verbindungen angereicherte wässrige Phase. Anschließend entfernt man die wässrige Phase. Das Inkontaktbringen kann kontinuierlich oder diskontinuierlich erfolgen.

Zur diskontinuierlichen Durchführung bringt man unter mechanischer Bewegung, z. B. durch Rühren, das an Isopulegol abgereicherte Sumpfprodukt aus Schritt a) und das wässrige Extraktionsmittel in einem geeigneten Gefäß in Kontakt, lässt das Gemisch zur Phasentrennung ruhen und entfernt eine der Phasen, indem man zweckmäßigerweise die dichtere Phase am Boden des Gefäßes abzieht.

Mehrere diskontinuierliche Trennoperationen können kaskadenartig hintereinander durchgeführt werden, wobei die von der wässrigen Phase abgetrennte, die Hauptmenge des Liganden der Formel (I) enthaltende Phase jeweils mit einer frischen Portion des wässrigen Extraktionsmittels in Kontakt gebracht wird und/oder das wässrige Extraktionsmittel im Gegenstrom geführt wird.

Vorzugsweise erfolgt die Extraktion kontinuierlich. Zur kontinuierlichen Durchführung der Extraktion führt man das wässrige Extraktionsmittel und den Strom des an Isopulegol abgereicherten Sumpfprodukts aus Schritt a) geeigneten Apparaturen in analoger Weise zur diskontinuierlichen Variante kontinuierlich zu. Gleichzeitig wird der Apparatur, in der die Trennung der Phasen stattfindet, ein Austrag der die Hauptmenge des Liganden der Formel (I) enthaltende Phase und ein Austrag der an Aluminium-haltigen Verbindungen angereicherte wässrige Phase kontinuierlich entnommen.

Die Extraktion erfolgt mindestens einstufig, z. B. in einer Mischer-Abscheider-Kombination. Geeignete Mischer sind sowohl dynamische als auch statische Mischer. Eine Extraktion in mehreren Stufen erfolgt beispielsweise in mehreren Mischer-Abscheidern oder Extraktionskolonnen.

In einer geeigneten Ausführungsform wird zur Verbesserung der Phasentrennung wenigstens eine Koalesziervorrichtung eingesetzt. Diese ist vorzugsweise ausgewählt unter Koaleszierfiltern, Elektrokoaleszern und Kombinationen davon. Beim Einsatz von Mischer-Abscheider-Vorrichtungen zur Extraktion hat sich der Einsatz von Koaleszierfiltern, wie Kerzen- oder Sandfiltern, als vorteilhaft zur Verbesserung der Phasentrennung herausgestellt. Der Filter kann dabei direkt nach dem Mischer (Rührbehälter) und/oder im organischen Ablauf des Abscheiders installiert werden. Des Weiteren bevorzugt zur Verbesserung der Phasentrennung ist der Einsatz von Elektrokoaleszern. Diese haben sich zur Abtrennung wässriger Fremdphasen von bis zu 5 Massen-% bewährt. Der Einsatz von Koalesziervorrichtungen eignet sich in dem erfindungsgemäßen Verfahren auch vorteilhaft zur Abscheidung von feindispergierter wässriger Phase aus dem die Hauptmenge des Liganden der Formel (I) enthaltenden organischen Austrag einer Extraktionskolonne.

In einer geeigneten Ausgestaltung erfolgt die Extraktion in wenigstens einer Mischer-Abscheider-Kombination für die Extraktion Aluminium-haltiger Komponenten aus dem an Isopulegol abgereicherten Sumpfprodukt aus Schritt a). Die Verwendung einer weiteren Mischer-Abscheider-Kombination ist insbesondere vorteilhaft, um Anteile des Liganden der Formel (I) oder gegebenenfalls des höhersiedenden Lösungsmittels, die gegebenenfalls mit den abzutrennenden Aluminium-haltigen Verbindungen teilweise in das Extraktionsmittel übergehen, nachträglich zu re-extrahieren und somit in das Verfahren zurückzuführen.

Unter bestimmten Umständen kann es vorteilhaft sein, die die Hauptmenge an Liganden der Formel (I) enthaltende organischen Phase vor dem Abtrennen des Liganden in Schritt c) oder im Anschluss an das Abtrennen einem Trocknungsschritt zu unterziehen. Geeignete Trocknungsverfahren sind die üblichen, dem Fachmann bekannten, insbesondere die Adsorption an wasserentziehenden Mitteln, z. B. unter Verwendung eines zeolithischen Molekularsiebs.

In einer alternativen Ausführungsform des erfindungsgemäßen Verfahrens wird, nach dem Inkontaktbringen des an Isopulegol abgereicherten Sumpfprodukts mit der wässrigen Base, das Wasser vollständig oder wenigstens teilweise destillativ entfernt.

Um ein frühzeitiges Abtrennen, speziell durch Kristallisation, des Liganden der Formel (I) zu verhindern, sollte zu keinem Zeitpunkt während Schritt b) das Löslichkeitsprodukt des Liganden in der organischen Phase überschritten werden. Dies kann durch geeignete Wahl der Temperatur und/oder der Menge und Art gegebenenfalls zugegebener Lösungsmittel erfolgen.

Demzufolge wird in einer bevorzugten Ausführungsform des erfindungsgemäßen Verfahrens ein Austrag des erwärmten Sumpfprodukts aus Schritt a) mit einer erwärmten wässrigen Base in innigen Kontakt gebracht.

Der Ausdruck "erwärmt" bezeichnet im Rahmen der vorliegenden Erfindung eine Temperatur oberhalb der Raumtemperatur und unterhalb der jeweiligen Siedepunktstemperaturen der wässrigen oder organischen Lösungen bei den jeweiligen Reaktionsbedingungen. Insbesondere bezeichnet erwärmt eine Temperatur im Bereich von 25 °C bis 150 °C, speziell im Bereich von 70 °C bis 100 °C.

In Abhängigkeit der gegebenenfalls bei der Cyclisierung von Citronellal verwendeten Hilfsstoffe kann das an Isopulegol abgereicherte Sumpfprodukt gegebenenfalls weitere in Schritt a) nicht abgetrennte Komponenten enthalten. Diese werden vorzugsweise in Schritt b) abgetrennt. In diesem Fall kann man die erhaltene wässrige Phase einem geeigneten Trennverfahren unterziehen, um diese Komponenten, z. B. einen Hilfsstoff (iv), zurückzugewinnen.

### Schritt c):

In Schritt c) des erfindungsgemäßen Verfahrens wird der Ligand der Formel (I) aus der in Schritt b) erhaltenen die Hauptmenge des Liganden enthaltenden organischen Phase durch Kristallisation abgetrennt, wobei Schritt c) kontinuierlich oder diskontinuierlich ausgeführt werden kann. Geeignete Ausführungsformen dieses Schritts sind beispielsweise Kristallisation und/oder vollständiges oder wenigstens teilweises destillatives Entfernen volatiler Bestandteile.

In einer bevorzugten Ausführungsform des erfindungsgemäßen Verfahrens erfolgt das Abtrennen des Liganden der Formel (I) durch Kristallisation.

Zur Kristallisation des Liganden der Formel (I) muss zunächst das Löslichkeitsprodukt des Liganden der Formel (I) in der organischen Phase aus Schritt b) überschritten werden. Dies kann beispielsweise durch einen Abkühlungsprozess der organischen Phase oder durch (teilweises) destillatives Abtrennen des Lösungsmittels erfolgen. Verfahren hierzu sind dem Fachmann bekannt. Zur technischen Ausgestaltung des erfindungsgemäßen Verfahrens sind beispielsweise übliche Kühlungskristallisatoren, Verdampfungskristallisatoren, Vakuumkristallisatoren, Kristallisierrinnen oder Sprühkristallisatoren geeignet.

In einer bevorzugten Ausführungsform des erfindungsgemäßen Verfahrens erfolgt die Kristallisation durch Abkühlen der organischen Phase aus Schritt b) des Verfahrens. Im Allgemeinen erfolgt die Kristallisation bei einer Temperatur im Bereich von -50 °C bis 100 °C, bevorzugt im Bereich von -20 °C bis 50 °C und speziell in einem Bereich von 10 °C bis 40 °C.

Dieser Prozess kann durch Hinzufügen von Impfkristallen beschleunigt werden.

Der kristalline Ligand der Formel (I) kann beispielsweise durch Filtration, Flotation, Zentrifugation oder Siebung aus der Lösung isoliert werden.

Der so zurückerhaltene Ligand der Formel (I) kann gegebenenfalls durch geeignete Trocknungsverfahren getrocknet werden. Verfahren hierfür sind dem Fachmann bekannt. Beispielsweise sind zur technischen Ausgestaltung des Verfahrens übliche Walzentrockner, Tellertrockner, Kammertrockner, Fließbetttrockner oder Strahlungstrockner geeignet.

Die an Ligand der Formel (I) abgereicherte organische Phase kann dem Verfahren erneut vor oder während Schritt a) zugeführt werden.

In einer geeigneten Ausführungsform des erfindungsgemäßen Verfahrens erfolgt die Kristallisation bei Abkühlen auf Raumtemperatur aus einer erwärmten, gesättigten, in Schritt b) erhaltenen organischen Phase.

In einer bevorzugten Ausführungsform des Verfahrens zur Aufarbeitung eines Reaktionsprodukts aus der Herstellung von Isopulegol ist der Ligand der Formel (I) ausgewählt unter Bis(diarylphenol)-Liganden der Formel (I.a) 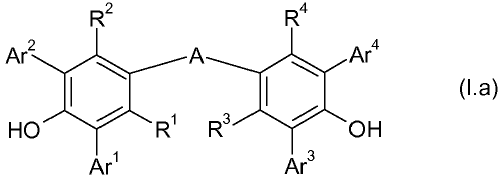 wobei Ar¹, Ar², Ar³, Ar⁴, R¹, R², R³, R⁴ und A die zuvor gegebenen Bedeutungen besitzen.

Die Liganden der Formel (I.a) weisen ebenfalls zwei Phenolsysteme auf, die jeweils in beiden ortho-Positionen zur phenolischen Hydroxy-Gruppe durch Aromaten bzw. Heteroaromaten (Ar¹ bis Ar⁴) substituiert sind und über ein Strukturelement A miteinander verknüpft sind und gegebenenfalls noch weitere Substituenten (R¹ bis R⁴) tragen können, wobei das Strukturelement A jeweils in para-Position zur phenolischen Hydroxy-Gruppe mit den beiden Phenolsystemen verknüpft ist. Dabei können den Resten Ar¹, Ar², Ar³, Ar⁴, den Resten R¹, R², R³, R⁴ und dem Strukturelement A die gleichen Bedeutungen zukommen wie vorstehend für Formel (I) genannt.

Insbesondere bevorzugte Liganden sind erfindungsgemäß solche, bei denen die Aryl-reste Ar¹, Ar², Ar³ und Ar⁴ gleich sind und die vorstehend für Formel (I) angegebenen bevorzugten Bedeutungen besitzen. Insbesondere bevorzugt als Arylreste Ar¹ bis Ar⁴ sind Phenyl, Naphthyl, 4-Fluorphenyl, 4-Chlorphenyl, 3-Chlorphenyl, 3,5-Dichlorphenyl, 4-Methylphenyl, 3-Trifluormethylphenyl, 4-Trifluormethylphenyl, ganz besonders bevorzugt Phenyl.

Bei den erfindungsgemäß bevorzugten Liganden der Formel (I.a) sind die Reste R¹, R², R³, R⁴ gleich oder verschieden, bevorzugt gleich, und bedeuten vorzugsweise: Wasserstoff, Halogen, insbesondere Fluor oder Chlor, Methyl, Trifluormethyl, Isopropyl, tert-Butyl, Phenyl, Nitro.

Dem Strukturelement A in Formel (I.a) kommen die vorstehend für Formel (I) genannten Bedeutungen zu. Bevorzugte Strukturelemente A in Formel (I.a) sind insbesondere auch die Strukturelemente 1 bis 44, die in der genannten Weise substituiert sein können.

Insbesondere bevorzugte Liganden sind solche der Formeln (I.a₁) bis (I.a₃), wobei den genannten Resten Ar¹ bis Ar⁴, R¹ bis R⁴ und R¹⁵ bis R¹⁸ bevorzugt die tabellarisch beispielhaft aufgeführten Bedeutungen zukommen:

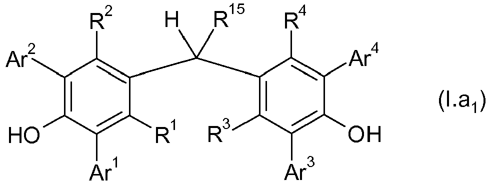

**Tabelle 1:**

| Verbindung | Ar¹ | Ar² | Ar³ | Ar⁴ | R¹ | R² | R³ | R⁴ | R¹⁵ |
|---|---|---|---|---|---|---|---|---|---|
| Ia₁-1 | Ph | Ph | Ph | Ph | H | H | H | H | H |
| Ia₁-2 | Ph | Ph | Ph | Ph | H | H | H | H | CH₃ |
| Ia₁-3 | Ph | Ph | Ph | Ph | H | H | H | H | Ph |
| Ia₁-4 | Ph | Ph | Ph | Ph | H | H | H | H | CF₃ |
| Ia₁-5 | Ph | Ph | Ph | Ph | H | H | H | H | CCl₃ |
| Ia₁-6 | Ph | Ph | Ph | Ph | H | H | H | H | 4-Cl-Ph |
| Ia₁-7 | Ph | Ph | Ph | Ph | H | H | H | H | CH₂CH₃ |
| Ia₁-8 | Ph | Ph | Ph | Ph | H | H | H | H | 3-NO₂-Ph |
| Ia₁-9 | Ph | Ph | Ph | Ph | H | H | H | H | 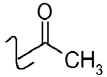 |

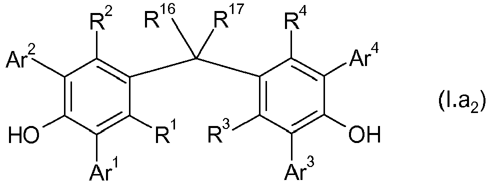

**Tabelle 2:**

| Verbindung | Ar¹ | Ar² | Ar³ | Ar⁴ | R¹ | R² | R³ | R⁴ | R¹⁶ | R¹⁷ |
|---|---|---|---|---|---|---|---|---|---|---|
| Ia₂-1 | Ph | Ph | Ph | Ph | H | H | H | H | CF₃ | CF₃ |
| Ia₂-2 | Ph | Ph | Ph | Ph | H | H | H | H | CCl₃ | CCl₃ |
| Ia₂-3 | Ph | Ph | Ph | Ph | H | H | H | H | CH₃ | CF₃ |
| Ia₂-4 | Ph | Ph | Ph | Ph | H | H | H | H | CH₃ | CCl₃ |
| Ia₂-5 | Ph | Ph | Ph | Ph | H | H | H | H | CH₂CH₃ | CF₃ |
| Ia₂-6 | Ph | Ph | Ph | Ph | H | H | H | H | CH₃ | CH₃ |
| Ia₂-7 | Ph | Ph | Ph | Ph | H | H | H | H | CH₃ | C(O)OCH₃ |
| Ia₂-8 | Ph | Ph | Ph | Ph | H | H | H | H | CH₃ | C(O)OC₂H₅ |
| Ia₂-9 | Ph | Ph | Ph | Ph | H | H | H | H | -(CH₂)₃- | |
| Ia₂-10 | Ph | Ph | Ph | Ph | H | H | H | H | -(CH₂)₄- | |
| Ia₂-11 | Ph | Ph | Ph | Ph | H | H | H | H | -(CH₂)₅- | |

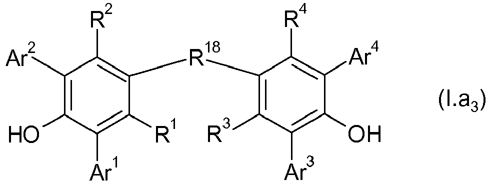

**Tabelle 3:**

| Verbindung | Ar¹ | Ar² | Ar³ | Ar⁴ | R¹ | R² | R³ | R⁴ | R¹⁸ |
|---|---|---|---|---|---|---|---|---|---|
| Ia₃-1 | Ph | Ph | Ph | Ph | H | H | H | H | -(CH₂)₂- |
| Ia₃-2 | Ph | Ph | Ph | Ph | H | H | H | H | 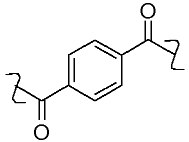 |
| Ia₃-3 | Ph | Ph | Ph | Ph | H | H | H | H | 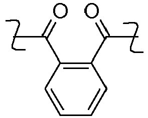 |
| Ia₃-4 | Ph | Ph | Ph | Ph | H | H | H | H | 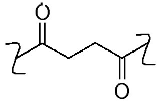 |
| Ia₃-5 | Ph | Ph | Ph | Ph | H | H | H | H | 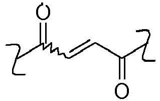 |
| Ia₃-6 | Ph | Ph | Ph | Ph | H | H | H | H | 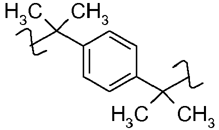 |
| Ia₃-7 | Ph | Ph | Ph | Ph | H | H | H | H | 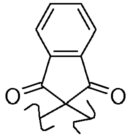 |

Dabei steht in den Tabellen 1 - 3 Ph für einen Phenylrest und C(O) steht im Rahmen der vorliegenden Erfindung für eine Carbonylgruppe. Generell können die Reste R¹⁵, R¹⁶ und R¹⁷ unabhängig voneinander für wie vorstehend definiertes C₁-C₆-Alkyl, C₁-C₁₀-Acyl, C₁-C₁₀-Carboxyl oder C₆-C₁₀-Aryl stehen, wobei die genannten Reste einen oder mehrere gleiche oder verschiedene Halogen- und/oder NO₂-Substituenten tragen können und wobei die Reste R¹⁶ und R¹⁷ gemeinsam auch ein cyclisches Strukturelement, bevorzugt eine Alkylenbrücke bilden können.

Ein weiterer Gegenstand der vorliegenden Erfindung betrifft ein Verfahren zur Herstellung von Isopulegol der Formel (IV) 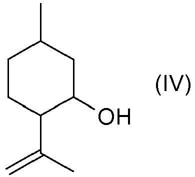 umfassend
α) die Cyclisierung von Citronellal der Formel (V) 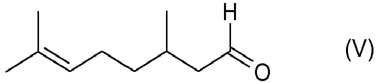 in Gegenwart eines Katalysators, der erhältlich ist durch Umsetzung eines Bis(diarylphenol)-Liganden der Formel (I), wie in den Ansprüchen 1 und/oder 10 definiert,
   mit einer Aluminium-Verbindung der Formel (II),

   (R¹⁴)₃₋ₚAlHₚ (II)

   wobei
   - Al: Aluminium bedeutet,
   - R¹⁴: einen verzweigten oder unverzweigten Alkylrest mit 1 bis 5 Kohlenstoffatomen und
   - p: für 0 oder eine ganze Zahl von 1 bis 3 steht,
   und/oder
   mit einer Aluminium-Verbindung der Formel (III),

   MAlH₄ (III)

   wobei
   - Al: Aluminium bedeutet und
   - M: Lithium, Natrium oder Kalium bedeutet,
β) die Rückgewinnung des Bis(diarylphenol)-Liganden der Formel (I) nach erfolgter Umsetzung, indem man
   a) das in Schritt α) erhaltene Aluminium-haltige Reaktionsprodukt einer destillativen Auftrennung unter Erhalt eines an Isopulegol angereicherten Kopfprodukts und eines an Isopulegol abgereicherten Sumpfprodukts unterzieht,
   b) das an Isopulegol abgereicherte Sumpfprodukt mit einer wässrigen Base unter Erhalt einer Aluminium-haltigen wässrigen Phase und einer die Hauptmenge der Liganden der Formel (I) enthaltenden organischen Phase in innigen Kontakt bringt und
   c) den Liganden der Formel (I) aus der organischen Phase abtrennt.

In einer speziellen Ausführungsform des erfindungsgemäßen Verfahrens zur Herstellung von Isopulegol erfolgt das Abtrennen des Liganden der Formel (I) durch Kristallisation.

Bezüglich der bevorzugten Ausführungsformen des erfindungsgemäßen Verfahrens zur Aufarbeitung eines Reaktionsprodukts aus der Herstellung von Isopulegol durch Cyclisierung von Citronellal sowie für die bevorzugten Liganden der Formel (I) wird im vollen Umfang auf die zuvor genannten bevorzugten Ausführungsformen verwiesen.

Die zur Herstellung der erfindungsgemäß verwendeten Bis(diarylphenoxy)-AluminiumVerbindungen einsetzbaren Bis(diarylphenol)-Liganden der Formeln (I) bzw. (I.a) lassen sich durch dem Fachmann an sich bekannte Methoden leicht herstellen. Verbindungen des Strukturtyps (I.a₁) erhält man beispielsweise durch Umsetzung der entsprechenden bis-ortho-Arylphenole mit einem Aldehyd R¹⁵CHO in Gegenwart einer Lewis-Säure, beispielsweise AlCl₃, wie u. a. beschrieben von Z. Y. Wang, A. S. Hay in Synthesis 1989, 471-472 oder in der US 3,739,035. Liganden des Strukturtyps (I.a₂) sind beispielsweise zugänglich durch Umsetzung der entsprechenden bis-ortho-Arylphenole mit einem geeigneten Keton der Formel R¹⁶C(O)R¹⁷, wie beispielsweise in der US 3,739,035 beschrieben. Liganden des Strukturtyps (I.a₃) sind beispielsweise zugänglich durch Friedel-Crafts-Acylierung der entsprechenden Phenole oder O-geschützten Phenole mit Dicarbonsäurechloriden wie beispielsweise von F. F. Blicke et al. in J. Am. Chem. Soc. 1938, 60, 2283-2285; der CH 350461 oder von G. Maier et al. in Chem. Ber. 1985, 118, 704-721 beschrieben. Eine weitere Möglichkeit zur Herstellung von Liganden des Strukturtyps (Ia₃) besteht auch in der Friedel-Crafts-Alkylierung der entsprechenden Phenole mit tertiären Diolen, wie beispielsweise in DE-A 25 34 558 beschrieben oder mit Dihalogeniden, wie beispielsweise von J. Zavada, in Collect. Czech. Chem. Commun., 1976, 41, 1777-1790, beschrieben.

Die erfindungsgemäß verwendeten Bis(diarylphenoxy)-Aluminium-Verbindungen erhält man beispielsweise, indem man die vorstehend beschriebenen Bis(diarylphenol)-Liganden der Formeln (I) bzw. (I.a) mit einer Aluminium-Verbindung der Formel (II)

(R¹⁴)₃₋ₚAlHₚ (II),

umsetzt. Dabei steht R¹⁴ für einen verzweigten oder unverzweigten Alkylrest mit 1 bis 5 Kohlenstoffatomen wie beispielsweise Methyl, Ethyl, Propyl, Isopropyl, Butyl, Isobutyl, Pentyl, Isopentyl oder Neopentyl. Der Index p steht für 0 oder eine ganze Zahl von 1 bis 3. Bevorzugt steht der Index p für 1 oder 0, besonders bevorzugt für 0. Bevorzugte Verbindungen der Formel (II) sind beispielsweise Trimethylaluminium, Triethylaluminium, Diisobutylaluminiumhydrid, besonders bevorzugt Trimethylaluminium und Triethylaluminium.

Alternativ dazu erhält man die erfindungsgemäß verwendeten Bis(diarylphenoxy)-Aluminium-Verbindungen auch durch Umsetzung der wie vorstehend beschriebenen Bis(diarylphenol)-Liganden der Formeln (I) bzw. (I.a) mit einer Aluminium-Verbindung der Formel (III)

MAlH₄ (III),

wobei M Lithium, Natrium oder Kalium bedeutet. Demzufolge eignen sich zur Herstellung der erfindungsgemäß verwendeten Bis(diarylphenoxy)-Aluminium-Verbindungen durch Umsetzung der wie vorstehend beschriebenen Bis(diarylphenol)-Liganden der Formeln (I) bzw. (I.a) auch Lithiumaluminiumhydrid, Natriumaluminiumhydrid und Kaliumaluminiumhydrid sowie Gemische derselben. Darüber hinaus sind auch Gemische der genannten Verbindungen der Formeln (II) und (III) zur Herstellung erfindungsgemäß verwendeten Bis(diarylphenoxy)-Aluminium-Verbindungen durch Umsetzung mit den wie vorstehend beschriebenen Bis(diarylphenol)-Liganden der Formeln (I) bzw. (I.a) geeignet.

Die Umsetzung wird vorteilhaft so durchgeführt, dass einer der wie vorstehend beschriebenen Bis(diarylphenol)-Liganden der Formeln (I) bzw. (I.a) mit einer Verbindung der Formel (II) oder (III) in Kontakt gebracht wird. Vorteilhaft führt man die Umsetzung in einem inerten organischen Lösungsmittel wie beispielsweise Toluol, Cyclohexan, Dichlormethan, Xylol, Ethylbenzol, Chlorbenzol, Tetrahydrofuran, Diethylether, Methyl-tert-Butylether, Essigsäureethylester, Pentan, Hexan, Dichlorethan, Dimethylformamid (DMF), Dimethylsulfoxid (DMSO) und dergleichen mehr durch, wobei der Einsatz vorgetrockneter bzw. wasserfreier Lösungsmittel als besonders vorteilhaft anzusehen ist. Üblicherweise erfolgt die Umsetzung bei Temperaturen im Bereich von etwa -100 °C bis etwa 100 °C, bevorzugt bei etwa -50 °C bis etwa 50 °C, besonders bevorzugt bei etwa -30 °C bis etwa 30 °C.

Bei der Herstellung der erfindungsgemäßen Bis(diarylphenoxy)-AluminiumVerbindungen reagieren die phenolischen Hydroxy-Gruppen der eingesetzten Bis(diarylphenol)-Liganden der Formeln (I) bzw. (I.a) mit der bzw. den Verbindungen der Formeln (II) und (III). Theoretisch kann jedes Aluminium-Atom mit 1 bis 3 phenolischen Hydroxy-Gruppen regieren. Aufgrund der sterischen Eigenschaften bzw. Anforderungen der eingesetzten Bis(diarylphenol)-Liganden der Formeln (I) bzw. (I.a) kann es dabei zur Ausbildung höhermolekularer Strukturen wie linearen Strukturen oder Netzwerken kommen.

Vorteilhaft wählt man dabei das molare Verhältnis der eingesetzten Bis(diarylphenol)-Liganden der Formeln (I) bzw. (I.a) zu den eingesetzten Verbindungen der Formel (II) und/oder (III) so, dass die Menge an nicht abreagierten Verbindungen der Formeln (II) und/oder (III) möglichst gering ist. Vorzugsweise wählt man das genannte Verhältnis so, dass nach dem Inkontaktbringen der Bis(diarylphenol)-Liganden der Formeln (I) bzw. (I.a) mit der bzw. den Verbindungen der Formeln (II) und (III) keine nicht umgesetzte Verbindung der Formel (II) und/oder (III) mehr vorliegt. Unter Berücksichtigung des wirtschaftlichen Aspekts ist es empfehlenswert, den Überschuss der eingesetzten Liganden der Formeln (I) bzw. (I.a) gering zu halten. Besonders bevorzugt setzt man Bis(diarylphenol)-Liganden der Formeln (I) bzw. (I.a) und die Verbindungen der Formeln (II) und/oder (III) in einem molaren Verhältnis von etwa 4:1 bis etwa 1:1, ganz besonders bevorzugt von etwa 3:1 bis etwa 1,5:1 und am meisten bevorzugt im molaren Verhältnis von etwa 1,5:1 ein.

Zur Herstellung der erfindungsgemäß verwendeten Bis(diarylphenoxy)-AluminiumVerbindungen geht man im Rahmen einer bevorzugten Ausführungsform der vorliegenden Erfindung so vor, das man, je nach Löslichkeit, eine etwa 0,001 bis etwa 1 molare Lösung des gewählten Liganden der Formel (I) bzw. (I.a) in einem geeigneten organischen Lösungsmittel, beispielsweise Toluol, bei einer Temperatur von etwa -10 bis etwa 30 °C vorlegt und eine Aluminiumverbindung der Formel (II) und/oder (III), vorzugsweise in Form einer Lösung, beispielsweise eine Lösung von Trimethyl- oder Triethylaluminium in Toluol, zugibt.

Die Reaktion zwischen den eingesetzten Liganden der Formel (I) bzw. (I.a) und den Aluminiumverbindungen der Formeln (II) und/oder (III) erfolgt in der Regel rasch und ist meist, in Abhängigkeit von den gewählten Reaktionsbedingungen, nach etwa 10 min bis etwa 2 h, oft nach etwa 1 h, abgeschlossen. Bei Einsatz reaktionsträgerer Reaktanden kann es vorteilhaft sein, die Temperatur des Reaktionsgemisches kurzzeitig zu erhöhen.

In Abhängigkeit von den gewählten Reaktionsbedingungen, insbesondere im Hinblick auf die Löslichkeit der umzusetzenden Liganden der Formel (I) bzw. (I.a) und der Aluminiumverbindung der Formel (II) und/oder (III) in den gewählten Lösungsmitteln, den Konzentrationen sowie den Reaktionstemperaturen, erhält man die erfindungsgemäßen Bis(diarylphenoxy)-Aluminium-Verbindungen in Form eines Feststoffes, einer Suspension oder einer Lösung im eingesetzten Lösungsmittel bzw. Lösungsmittelgemisch. Die so erhaltenen erfindungsgemäß verwendeten Bis(diarylphenoxy)-AluminiumVerbindungen können in der jeweils erhaltenen Form weiterverwendet werden oder abgetrennt und von den eingesetzten Lösungsmitteln befreit werden.

Die Isolierung kann dabei nach dem Fachmann bekannten und vorteilhaft erscheinenden Methoden erfolgen. Vorzugsweise führt man die Isolierung, Lagerung bzw. Weiterbehandlung der erfindungsgemäß verwendeten Bis(diarylphenoxy)-AluminiumVerbindungen unter weitgehendem Ausschluss von Sauerstoff und Feuchtigkeit durch.

Zur Durchführung des erfindungsgemäßen Verfahrens zur Herstellung von Isopulegol geht man vorteilhaft so vor, dass man zunächst eine Lösung der erfindungsgemäß verwendeten Bis(diarylphenoxy)-Aluminium-Verbindungen in einem geeigneten Lösungsmittel, wie vorstehend beschrieben, bereitstellt. Dieser Lösung setzt man dann erfindungsgemäß das zu cyclisierende racemische oder nicht-racemische Citronellal zu. Das Citronellal kann dabei als solches oder in Form einer Lösung, vorteilhaft in einem der vorstehend genannten geeigneten Lösungsmittel zugegeben werden. Im Rahmen einer bevorzugten Ausführungsform des erfindungsgemäßen Verfahrens bereitet man zunächst eine Lösung des gewählten Liganden der Formeln (I) bzw. (I.a) in Toluol und setzt dann, vorteilhaft unter Rühren, die gewählte Aluminium-Verbindung der Formel (II) und/oder (III), bevorzugt Trimethyl- oder Triethylaluminium in toluolischer, Lösung zu.

Als Ausgangsstoff zur Durchführung des erfindungsgemäßen Cyclisierungsverfahrens eignet sich Citronellal, das nach jedwedem Verfahren hergestellt sein kann. Bevorzugt setzt man Citronellal ein, das eine Reinheit von etwa 90 bis etwa 99,9 Gew.-%, besonders bevorzugt von etwa 95 bis etwa 99,9 Gew.-%, aufweist.

Die Zugabe des zu cyclisierenden Citronellals erfolgt vorteilhaft bei Temperaturen im Bereich von etwa -40 °C bis etwa 40 °C, bevorzugt im Bereich von etwa -20 °C bis etwa 20 °C. Dazu wird vorteilhaft die bereitete Lösung der erfindungsgemäß verwendeten Bis(diarylphenoxy)-Aluminium-Verbindung auf eine Temperatur in diesem Bereich, z. B. auf eine Temperatur im Bereich von -10 °C bis 10 °C abgekühlt und vorgekühltes Citronellal bzw. eine vorgekühlte Lösung von Citronellal zugegeben.

Die Zugabe des Citronellals bzw. der Lösung davon kann so vorgenommen werden, dass man entweder die gesamte Menge auf einmal zusetzt oder sie portionsweise oder auch kontinuierlich zur bereiteten Katalysatorlösung gibt. Als Lösungsmittel eignen sich wiederum die vorstehend genannten Lösungsmittel, insbesondere Toluol. Bevorzugt setzt man das zu cyclisierende Citronellal als solches, d. h. ohne weiteren Zusatz von Lösungsmitteln, ein. Bei Einsatz eines Lösungsmittels wird die gesamte Lösungsmittelmenge (für Katalysatorherstellung und zur Durchführung der Cyclisierungsreaktion) vorteilhaft so gewählt, dass das volumenbezogene Verhältnis von umzusetzendem Citronellal zum Lösungsmittel etwa 2:1 bis etwa 1:20, bevorzugt von etwa 1,5:1 bis etwa 1:10 beträgt.

Das Mengenverhältnis zwischen dem umzusetzendem Citronellal und der eingesetzten Menge der erfindungsgemäß verwendeten Bis(diarylphenoxy)-Aluminium-Verbindung wird durch die Menge der zur Herstellung derselben eingesetzten Verbindungen der Formel (I) bzw. (I.a) und der Formel (II) und/oder (III), also durch das Mengenverhältnis von eingesetztem Ligand zu eingesetzter Aluminium-Verbindung der Formel (II) und/oder (III) bestimmt.

Erfindungsgemäß wählt man die Menge von umzusetzendem Citronellal zur eingesetzten Menge an Aluminium-Verbindung der Formel (II) und/oder (III) so, dass das molare Verhältnis etwa 5:1 bis etwa 1000:1, bevorzugt etwa 10:1 bis etwa 500:1, besonders bevorzugt etwa 50:1 bis etwa 200:1 beträgt.

Unabhängig davon kann das Verhältnis zwischen eingesetztem Ligand der Formel (I) bzw. (I.a) und der eingesetzten Aluminium-Verbindung der Formel (II) und/oder (III) in den vorstehend zur Herstellung der erfindungsgemäßen Bis(diarylphenoxy)-Aluminium-Verbindung genannten Grenzen variiert werden.

Die Cyclisierung von Citronellal zu Isopulegol erfolgt je nach Wahl der Reaktionspartner und Reaktionsbedingungen in der Regel rasch und ist üblicherweise nach etwa 0,5 bis etwa 10 h, oft nach etwa 5 h, weitgehend abgeschlossen. Der Reaktionsfortschritt kann durch dem Fachmann an sich bekannte Methoden, beispielsweise durch chromatographische, speziell gaschromatographische Methoden oder auch HPLC-Methoden leicht verfolgt werden.

Im Rahmen einer bevorzugten Ausführungsform des erfindungsgemäßen Verfahrens führt man die Cyclisierung von Citronellal zu Isopulegol in Gegenwart eines Hilfsstoffs (iv), beispielsweise einer Säure, vorzugsweise einer organischen Säure durch. Beispielhaft seien als vorteilhaft einsetzbare organische Säuren genannt: Essigsäure, Propionsäure, Benzoesäure, Toluolsulfonsäure, Methansulfonsäure, bevorzugt Essigsäure. Die genannten Säuren werden vorteilhaft in einer Menge von etwa 0,5 bis etwa 10 Gew.-%, bezogen auf die Menge an umzusetzendem Citronellal, eingesetzt. Vorteilhaft werden sie zusammen mit dem Citronellal, z. B. in Form eines Gemisches, dem Reaktionsgemisch zugesetzt.

In einer besonders bevorzugten Ausführungsform führt man das erfindungsgemäße Verfahren zur Herstellung von Isopulegol durch Cyclisierung von Citronellal in Gegenwart mindestens eines Hilfsstoffs (iv) durch, der ausgewählt ist unter Carbonsäureanhydriden, Aldehyden, Ketonen und Vinylethern.

Die Hilfsstoffe (iv) der genannten Substanzklassen können jeweils einzeln oder in Form von Gemischen untereinander eingesetzt werden. Bevorzugt setzt man im Fall von Gemischen solche ein, die aus Verbindungen einer Substanzklasse bestehen. Besonders bevorzugt setzt man einzelne Verbindungen ein. Unter wie nachfolgend beschriebenem Einsatz der genannten Verbindungen gelingt es in der Regel, die Bildung unerwünschter Nebenprodukte weitgehend zu unterdrücken.

Im Rahmen einer bevorzugten Ausführungsform führt man die Cyclisierung von Citronellal in Gegenwart eines Carbonsäureanhydrids der Formel (VI) 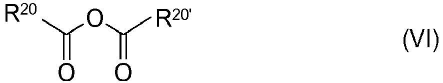 durch wobei die Reste R²⁰ und R^{20'} gleich oder verschieden, bevorzugt gleich, sein können und einen verzweigten oder unverzweigten C₁-C₁₂-Alkylrest oder C₇-C₁₂-Aralkylrest oder einen C₆-C₁₀-Arylrest bedeuten, wobei die genannten Reste jeweils einen oder mehrere, in der Regel 1 bis etwa 3, gleiche oder verschiedene Substituenten, ausgewählt aus der Gruppe OR^{10e}, SR^{10f} NR^{8e}R^{9e} und Halogen aufweisen können und wobei R²⁰ und R^{20'} gemeinsam auch einen 5- bis 8-gliedrigen Ring bilden können, der eine oder mehrere ethylenische Doppelbindungen und ein oder mehrere gleiche oder verschiedene Heteroatome, ausgewählt aus der Gruppe O, S und NR^{11b} aufweisen kann und wobei R^{10e}, R^{10f}, R^{8e}, R^{9e} und R^{11b} die vorstehend für R¹¹ angegebenen Bedeutungen haben können.

Im Rahmen einer weiteren bevorzugten Ausführungsform führt man die Cyclisierung von Citronellal in Gegenwart eines Aldehyds der Formel (VII) 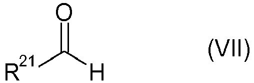 durch, wobei der Rest R²¹ einen verzweigten oder unverzweigten C₁-C₁₂-Alkylrest oder C₇-C₁₂-Aralkylrest oder einen C₆-C₁₀-Arylrest bedeutet, wobei die genannten Reste jeweils einen oder mehrere, bevorzugt 1 bis 3, gleiche oder verschiedene Substituenten, ausgewählt aus der Gruppe OR^{10e}, SR^{10f} NR^{8e}R^{9e} und Halogen aufweisen können, wobei R^{10e}, R^{10f}, R^{8e} und R^{9e} die vorstehend für R¹¹ angegebenen Bedeutungen haben können.

Im Rahmen einer weiteren bevorzugten Ausführungsform führt man Cyclisierung von Citronellal in Gegenwart eines Ketons der Formel (VIII) 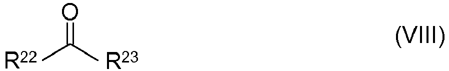 durch, wobei die Reste R²² und R²³ jeweils gleich oder verschieden sein können und einen verzweigten oder unverzweigten C₁-C₁₂-Alkylrest oder C₇-C₁₂-Aralkylrest oder einen C₆-C₁₀-Arylrest oder einen C₁-C₆-Alkoxycarbonylrest bedeuten, wobei die genannten Reste jeweils einen oder mehrere, bevorzugt 1 bis 3, gleiche oder verschiedene Substituenten, ausgewählt aus der Gruppe OR^{10e}, SR^{10f} NR^{8e}R^{9e} und Halogen aufweisen können, und wobei R²² und R²³ gemeinsam auch einen 5- bis 8-gliedrigen Ring bilden können, der eine oder mehrere ethylenische Doppelbindungen und ein oder mehrere gleiche oder verschiedene Heteroatome, ausgewählt aus der Gruppe O, S, NR^{11b} aufweisen kann und wobei R^{10e}, R^{10f}, R^{8e}, R^{9e} und R^{11b} die vorstehend für R¹¹ angegebenen Bedeutungen haben können.

Alternativ zu den zuvor genannten Carbonylverbindungen lassen sich auch Vinylether der allgemeinen Formel (IX) 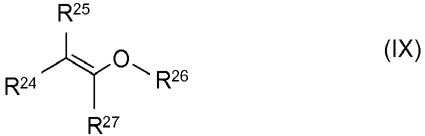 im Rahmen des erfindungsgemäßen Verfahrens einsetzen, wobei die Reste R²⁴, R²⁵, R²⁶ und R²⁷ unabhängig voneinander jeweils gleich oder verschieden sein können und einen verzweigten oder unverzweigten C₁-C₁₂-Alkylrest oder C₇-C₁₂-Aralkylrest oder einen C₆-C₁₀-Arylrest bedeuten, wobei die genannten Reste jeweils einen oder mehrere, bevorzugt 1 bis 3, gleiche oder verschiedene Substituenten, ausgewählt unter Oxo, OR^{10e}, SR^{10f} NR^{8e}R^{9e} und Halogen aufweisen können und wobei R²⁵ und R²⁶ gemeinsam auch einen 5- bis 8-gliedrigen Ring bilden können, der eine oder mehrere ethylenische Doppelbindungen und ein oder mehrere, üblicherweise 1 oder 2, gleiche oder verschiedene Heteroatome, ausgewählt aus der Gruppe O, S, NR^{11b} aufweisen kann und wobei R^{10e}, R^{10f}, R^{8e}, R^{9e} und R^{11b} die vorstehend für R¹¹ angegebenen Bedeutungen haben können.

C₁-C₁₂-Alkyl steht dabei für wie vorstehend beschriebenes C₁-C₆-Alkyl und darüber hinaus beispielsweise für Heptyl, Octyl, Nonyl, Decyl, Undecyl oder Dodecyl. In den Fällen, in denen zwei Alkylreste gemeinsam einen Ring ausbilden, sind unter Alkylresten auch Alkylenylreste zu verstehen. C₇-C₁₂-Aralkylresten und C₆-C₁₀-Arylresten können beispielhaft die vorstehend genannten Bedeutungen zukommen. Beispielhaft seien als C₁-C₆-Alkoxycarbonylrest genannt: Methoxycarbonyl, Ethoxycarbonyl, Propoxycarbonyl und Isopropoxycarbonyl, bevorzugt Methoxycarbonyl und Ethoxycarbonyl.

Im Rahmen einer bevorzugten Ausführungsform des erfindungsgemäßen Verfahrens führt man die Cyclisierung von Citronellal in Gegenwart eines Carbonsäureanhydrids der Formel (VI) durch, wobei die Reste R²⁰ und R^{20'} gleich sind und einen verzweigten oder unverzweigten C₁-C₁₂-Alkylrest oder C₇-C₁₂-Aralkylrest oder einen C₆-C₁₀-Arylrest bedeuten, und wobei R²⁰ und R^{20'} gemeinsam auch einen 5- bis 8-gliedrigen Ring bilden können, der eine oder mehrere ethylenische Doppelbindungen und ein oder mehrere gleiche oder verschiedene Heteroatome, ausgewählt aus der Gruppe OR^{10e}, SR^{10f}, NR^{11b} aufweisen kann und R^{10e}, R^{10f} und R^{11b} unabhängig voneinander die vorstehend für R¹¹ angegebenen Bedeutungen haben kann.

Insbesondere bevorzugt setzt man solche Carbonsäureanhydride ein, bei denen die Reste R²⁰ und R^{20'} gleich sind und einen verzweigten oder unverzweigten C₁-C₁₂-Alkylrest oder einen C₆-C₁₀-Arylrest bedeuten. Beispielhaft seien als erfindungsgemäß besonders bevorzugt einzusetzende Carbonsäureanhydride genannt: Essigsäureanhydrid, Propionsäureanhydrid, Pivalinsäureanhydrid und Benzoesäureanhydrid.

Als erfindungsgemäß ebenfalls bevorzugt einsetzbare Aldehyde der Formel (VII) seien beispielhaft Acetaldeyhd, Propionaldehyd und Chloral (Trichloracetaldehyd) genannt.

Führt man die Cyclisierung von Citronellal im Rahmen einer weiteren bevorzugten Ausführungsform in Gegenwart eines Ketons der Formel (VIII) durch, setzt man mit Vorteil solche mit einer aktivierten, d. h. elektronenarmen, Carbonylfunktion ein. Beispielhaft seien die folgenden Ketone genannten, die sich in besonderem Maße zum Einsatz im Rahmen des erfindungsgemäßen Verfahrens eigenen: 1,1,1-Trifluoraceton, 1,1,1-Trifluoracetophenon, Hexafluoraceton, Brenztraubensäuremethylester und Brenztraubensäureethylester.

Als erfindungsgemäß ebenfalls bevorzugt einsetzbare Vinylether der Formel (IX) seien beispielhaft genannt: Methylvinylether, Ethylvinylether, Isobutylvinylether und 3,4-Dihydro-2H-pyran.

Die genannten Verbindungsklassen können gleichermaßen mit gutem Erfolg im Rahmen dieser bevorzugten Ausführungsform des erfindungsgemäßen Verfahrens eingesetzt werden. Im Hinblick auf praktische Gesichtspunkte wie beispielsweise eine höhere Reaktionsgeschwindigkeit hat sich der Einsatz von Aldehyden und/oder elektronenarmen Ketonen als vorteilhaft erweisen.

Die Menge an erfindungsgemäß zu verwendendem Carbonsäureanhydrid, Aldehyd, Keton und/oder Vinylether kann in breiten Grenzen variiert werden und richtet sich nach Art des eingesetzten Stoffes und dem Reinheitsgrad bzw. dem Vorhandensein noch nicht näher identifizierter Verunreinigungen. Üblicherweise setzt man die genannten Verbindungen bzw. deren Gemische in einer Menge von etwa 0,01 mol-% bis etwa 5 mol-%, bevorzugt von etwa 0,1 mol-% bis etwa 2 mol-%, bezogen auf die eingesetzte Menge an Citronellal, ein.

An die Art und Weise der Reaktionsführung, beispielsweise die Ausgestaltung von Reaktoren oder die Reihenfolge der Zugabe einzelner Reaktionspartner, sind keine besonderen Anforderungen zu stellen, solange eine Reaktionsführung unter weitgehendem Ausschluss von Sauerstoff und Wasser gewährleistet ist.

Zur Durchführung des erfindungsgemäßen Verfahrens im Rahmen dieser bevorzugten Ausführungsform geht man vorteilhaft so vor, dass man zunächst eine Lösung der erfindungsgemäß einzusetzenden Bis(diarylphenoxy)-Aluminium-Verbindung in einem geeigneten Lösungsmittel wie vorstehend beschrieben bereitstellt. Dieser Lösung setzt man dann erfindungsgemäß bevorzugt ein Gemisch des zu cyclisierenden racemischen oder nicht-racemischen Citronellals mit dem gewählten Carbonsäureanhydrid, dem Aldehyd, dem aktivierten Keton und/oder dem Vinylether zu. Alternativ dazu kann man beispielsweise auch die Lösung der erfindungsgemäß einzusetzenden Bis(diaryl-phenoxy)-Aluminium-Verbindung zunächst mit dem gegebenenfalls jeweils gewählten Carbonsäureanhydrid, dem Aldehyd, dem Keton und/oder dem Vinylether versetzen und im Anschluss daran das zu cyclisierende Citronellal zugeben.

Es sich als vorteilhaft erwiesen, das Citronellal bzw. das Gemisch von Citronellal mit der gewählten Verbindung innerhalb eines Zeitraums von etwa 30 min bis etwa 6 h, bevorzugt innerhalb von etwa 2 h bis etwa 4 h, zur Katalysatorlösung bzw. dem Reaktionsgemisch zuzudosieren. Das Citronellal kann dabei als solches oder in Form einer Lösung, vorteilhaft in einem der vorstehend genannten geeigneten Lösungsmittel, zugegeben werden. Im Rahmen einer wiederum bevorzugten Ausführungsform des erfindungsgemäßen Verfahrens bereitet man zunächst eine Lösung des gewählten Liganden der Formeln (I) bzw. (I.a) in Toluol und setzt dann, zweckmäßigerweise unter Rühren, die gewählte Aluminium-Verbindung der Formel (II) und/oder (III), bevorzugt Trimethyl- oder Triethylaluminium in toluolischer Lösung zu.

Die Zugabe des zu cyclisierenden Citronellals bzw. des Gemischs von Citronellal mit dem gewählten Carbonsäureanhydrid, Aldehyd, aktivierten Keton und/oder dem Vinylether erfolgt im Rahmen dieser Ausführungsform vorteilhaft bei Temperaturen im Bereich von etwa -40 °C bis etwa 40 °C, bevorzugt im Bereich von etwa -20 °C bis etwa 20 °C. Dazu wird vorteilhaft die bereitete Lösung oder Suspension der erfindungsgemäßen Bis(diarylphenoxy)-Aluminium-Verbindung auf eine Temperatur in diesem Bereich, z. B. auf eine Temperatur im Bereich von -10 °C bis 10 °C, abgekühlt und die weiteren Reaktanden in vorgekühlter Form zugegeben.

Die Zugabe des Gemischs von Citronellal und der gewählten weiteren Verbindung kann so vorgenommen werden, dass man entweder die gesamte Menge Citronellal auf einmal zusetzt oder sie portionsweise oder auch kontinuierlich zur bereiteten Katalysatorlösung gibt. Als Lösungsmittel eignen sich wiederum bevorzugt die vorstehend genannten Lösungsmittel, insbesondere Toluol. Bevorzugt setzt man das zu cyclisierende Citronellal in Form eines Gemisches mit dem gewählten Carbonsäureanhydrid, Aldehyd, aktivierten Keton und/oder Vinylether ohne weiteren Zusatz von Lösungsmitteln ein. Bei Einsatz eines Lösungsmittels wählt man die gesamte Lösungsmittelmenge vorteilhaft so, dass das volumenbezogene Verhältnis von umzusetzendem Citronellal zum Lösungsmittel etwa 1 : 1 bis etwa 1 : 20, bevorzugt von etwa 1 : 1 bis etwa 1:10 beträgt.

Es wurde gefunden, dass üblicherweise während der Reaktion ein Teil des Katalysatorkomplexes deaktiviert wird. Dies ist unter anderem auf Ligandenaustausch-Prozesse zwischen den jeweils eingesetzten Bis(diarylphenol)-Liganden der Formel der eingesetzten Bis(diarylphenoxy)-Aluminium-Verbindungen und dem durch Cyclisierung entstehenden Isopulegol zurückzuführen. Die deaktivierte Form des Katalysators ist, in Abhängigkeit von der Wahl der eingesetzten Lösemittel, üblicherweise im Gegensatz zum aktiven polymeren Katalysator in der Reaktionsmischung löslich.

In einer bevorzugten Ausführungsform kann durch einfache physikalische Trennmethoden (z. B. durch Abfiltration oder Zentrifugation des noch aktiven Katalysators) der deaktiverte Teil des Katalysators zusammen mit der übrigen Reaktionsmischung abgetrennt werden. Der zurückgehaltene, immer noch aktive Teil des Katalysators kann gewünschtenfalls mit frischem Katalysator ergänzt werden und ohne nennenswerten Aktivitätsverlust wieder eingesetzt werden, vorzugsweise im Rahmen einer weiteren erfindungsgemäßen Cyclisierungsreaktion von Citronellal zu Isopulegol.

Alternativ kann die eingesetzte Katalysatormenge so gewählt werden, dass der gesamte eingesetzte Katalysatorkomplex im Laufe bzw. nach Beendigung der erfindungsgemäßen Cyclisierungsreaktion deaktiviert und somit löslich ist, was an einer klaren Reaktionsmischung zu erkennen ist. Dabei macht sich vorteilhaft bemerkbar, dass in diesem Falle aufgrund der vorstehend beschriebenen Ligandenaustausch-Prozesse der jeweils eingesetzte Bis(diarylphenol)-Ligand der Formel (I) ohne gesondert durchzuführende Hydrolyse freigesetzt wird.

Überraschenderweise wurde gefunden, dass Isopulegol aus den Aluminium-haltigen Reaktionsprodukten der Cyclisierung von Citronellal ohne vorherige Hydrolyse der jeweils als Katalysator eingesetzten (bis(Diarylphenoxy)-Aluminium-Verbindungen (gegebenenfalls nach der destillativen Entfernung eines eingesetzten Lösungsmittels und/oder zusätzlich eingesetzter Hilfsstoffe) in hohen Reinheiten abdestilliert werden kann. Dabei bilden sich im Destillationssumpf in der Regel keine erkennbaren unerwünschten oder störenden Nebenprodukte. In einer speziellen Ausführung erfolgt vor oder während der destillativen Auftrennung in Schritt a) die Zugabe eines geeigneten, inerten, hochsiedenden Lösungsmittels. Dann erhält man im Destillationssumpf eine Lösung des Liganden der Formel (I) in dem erwärmten, jeweils eingesetzten Hochsieder.

Das erfindungsgemäße Verfahren eignet sich, wie bereits erwähnt, in gleichem Maße zur Cyclisierung von racemischem wie auch nicht-racemischem, d. h. optisch aktivem Citronellal zu racemischem wie nicht-racemischem Isopulegol.

Daher dient das erfindungsgemäße Verfahren in einer bevorzugten Ausführungsform zur Herstellung von optisch aktivem Isopulegol der Formel (IV.a) 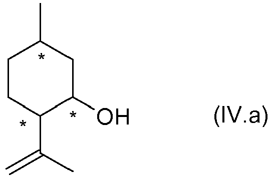 durch Cyclisierung von aktivem Citronellal der Formel (V.a) 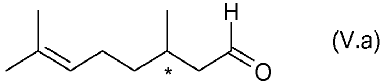 wobei (*) jeweils ein asymmetrisches Kohlenstoffatom bezeichnet.

Insbesondere dient das erfindungsgemäße Verfahren zur Herstellung von L-(-)-Ispulegol durch Cyclisierung von D-(+)-Citronellal.

Das so hergestellte racemische bzw. nicht-racemische Isopulegol stellt ein wertvolles Intermediat zur Herstellung von racemischem bzw. nicht-racemischem Menthol, einem der weltweit bedeutendsten Riech- bzw. Aromastoffe dar. Menthol kann durch dem Fachmann an sich bekannte Methoden der Hydrierung, speziell der katalytischen Hydrierung an geeigneten Übergangsmetallkatalysatoren, wie beispielsweise in Pickard et al., J. Chem. Soc. 1920, 1253; Ohloff et al., Chem. Ber. 1962, 95, 1400; Pavia et al., Bull. Soc. Chim. Fr. 1981, 24, Otsuka et al., Synthesis 1991, 665 oder in der EP 1 053 974 A beschrieben, aus Isopulegol erhalten werden. Dabei bleibt bei geeigneter Wahl der Reaktionsbedingungen die relative bzw. absolute Konfiguration des eingesetzten Isopulegols weitgehend, in vielen Fällen vollständig erhalten.

Ein weiterer Gegenstand der vorliegenden Erfindung betrifft daher ein Verfahren zur Herstellung von Menthol, umfassend die Schritte:
A) Herstellung von Isopulegol der Formel (IV) nach einem erfindungsgemäßen Verfahren
B) Hydrierung der ethylenischen Doppelbindung des so erhaltenen Isopulegols.

In einer bevorzugten Ausführungsform dient dieses Verfahren zur Herstellung von optisch aktivem Menthol, speziell zur Herstellung von L-(-)-Menthol aus optisch aktivem L-(-)-Isopulegol.

Bezüglich der bevorzugten Ausführungsformen des erfindungsgemäßen Verfahrens zur Herstellung von Isopulegol wird im vollen Umfang auf die zuvor genannten Bevorzugungen verwiesen.

Die nachfolgenden Beispiele dienen dazu, die vorliegende Erfindung zu erläutern.

### Beispiel 1: Verfahren zur Rückgewinnung von 1,1-Bis(2,6-diphenylphenol)-1-trifluormethylethan (la₂-3)

Gaschromatographische Analysen (GC) wurden nach folgender Methode durchgeführt: 50 m CP-WAX, ID.: 0,32 mm, FD.: 1,2 ym; 80 °C, 3 °C/min - 200 °C, 15 °C/min auf 250 °C; t_{R} (Phenylcyclohexan): 30,7; t_{R} (Isopulegol): 26,3; t_{R} (Citronellal): 21,8. Folgende HPLC-Methode wurde verwendet: CC250/4 Nucleodur C18 Gravity, 5 ym; C: Wasser - 0.,05 % H₃P0₄; D: Acetonitril 20 : 80; Ausgang: 93 bar, 25 °C; t_{R} (Phenylcyclohexan): 10,5; t_{R} (Isopulegol): 3,3; t_{R} (Ligand (Ia₂-3)): 14,0. Konzentrationen der erhaltenen Reaktionsprodukte im Destillationssumpf und in der Mutterlauge (jeweils in Gew.-%) wurden GC- und HPLC-analytisch mittels internem Standard ermittelt.

### 1.a) Cyclisierung von Citronellal

In einem Doppelmantelglasreaktor mit Rührer wurde 1,1-Bis(2,6-diphenylphenol)-1-trifluormethylethan (la₂-3) (461 g, 0,785 mol) in wasserfreiem Toluol (7,2 1) vorgelegt. Zu der klaren Lösung des Liganden wurde bei Raumtemperatur eine Lösung von Triethylaluminium in Toluol (445 ml, 400 mmol, 12 % AlEt₃ in Toluol) gegeben. Die Lösung wurde für 1 h bei 25 °C gerührt. Die resultierende Katalysator-Suspension wurde auf 0 °C gekühlt und über einen Zeitraum von 3 h mit einem Gemisch aus Citronellal (6697 g, 43 mol) und Brenztraubensäuremethylester (33,6 g, 329 mmol) versetzt. Nach beendeter Zugabe wurde die Reaktionsmischung 3 h bei 0 °C und weitere 2 h bei 10 °C nachgerührt. Toluol wurde unter vermindertem Druck abgetrennt. Anschließend wurde ein Isopulegol-Rohprodukt destillativ als Kopfprodukt unter Zugabe von Phenylcyclohexan (2770 g) abgetrennt. Dabei wurden 3584 g eines Sumpfprodukts erhalten.

### 1.b) Isolierung des Liganden (Ia₂-3)

In einem Doppelmantelreaktor mit Rührer und Rückflusskühler wurden 3564 g des Sumpfprodukts aus der Cyclisierung von Citronellal in Gegenwart eines (Bis(diarylphenoxy))-Aluminium-Katalysators, enthaltend Phenylcyclohexan (69,9 Gew.-%), Isopulegol (3,05 Gew.-%) Citronellal (0,16°Gew.-%) und Citronellol (3,05 Gew.-%), bei einer Temperatur von 90 °C vorgelegt. Zu der erwärmten Lösung wurden 1792 g einer erwärmten 2%igen NaOH-Lösung gegeben. Nach einstündigem Rühren bei 90 °C wurden 1777 g der wässrigen Phase von der organischen Phase abgetrennt. Das restliche Wasser aus der organischen Phase wurde bei 120 °C und 10 mbar abdestilliert. Das hydrolysierte Sumpfprodukt wurde innerhalb von 12 Stunden auf 25 °C abgekühlt. Die erhaltene Suspension des Liganden der Formel (la₂-3) wurde filtriert und der so gewonnene Ligand der Formel (la₂-3) wurde bei 3 mbar und 95 °C von volatilen Bestandteilen befreit. Der Ligand der Formel (la₂-3) wurde als weißer Feststoff mit einer Ausbeute von 282 g und einer Reinheit von 95 % isoliert. Die Mutterlauge (3130 g) enthielt laut HPLC-Analytik Phenylcyclohexan (72,3 Gew.-%), Isopulegol (6,8 Gew.-%) und Ligand der Formel (la₂-3) (4,9 Gew.-%). Dies belegt, dass sich die erfindungsgemäß verwendeten Liganden in vorteilhafter Weise für eine kontinuierliche Aufarbeitung eignen. Mit den in der EP-A 1 225 163 beschriebenen Liganden hingegen ist eine Trennung der Phasen nicht in jedem Fall gewährleistet, da diese in stärkerem Maße zur Bildung stabiler Emulsionen neigen.

### Beispiel 2: Kontinuierliches Verfahren zur Rückgewinnung von 1,1-Bis(2,6-diphenyl-phenol)-1-trifluormethylethan (Ia₂-3)

### Analytik

Gaschromatographische Analysen wurden nach der folgenden Methode durchgeführt: 50 m CP-WAX, ID.: 0,32 mm, FD.: 1,2 ym; 80 °C, 3 °C/min - 200 °C, 15 °C/min auf 250 °C; t_{R} (Citronellal): 20,7; t_{R} (Isopulegol): 24,7; t_{R} (Phenylcyclohexan): 29,3; t_{R} (Citronellol): 31,7; t_{R} (Citronellsäurecitronellylester): 48,2; t_{R} (Citronellsäure-isopulegylester): 49,5.

### 2.a) Cyclisierung von Citronellal bei kontinuierlicher Aufarbeitung

In einem Doppelmantelglasreaktor mit Rührer wird zu einer klaren Lösung von 1,1-Bis(2,6-diphenylphenol)-1-trifluormethylethan (la₂-3) (114 g, 0,195 mol) in Toluol (wasserfrei, 1800 g) bei 20 °C eine Lösung von Triethylaluminium in Toluol (15%ig, 85 ml, 0,096 mol) innerhalb von etwa 10 min zugegeben. Anschließend wird die Lösung 1 h bei 20 °C gerührt. Die resultierende Katalysator-Suspension wird in einen weiteren Doppelmantelglasreaktor mit Rührer überführt, auf 0 °C abgekühlt und über einen Zeitraum von 3 h mit einem Gemisch aus D-Citronellal (1620 g, 10,3 mol) und Brenztraubensäuremethylester (8,1 g) versetzt. Nach beendeter Zugabe wird die Reaktionslösung bei 0 °C gerührt bis ein Gehalt von < 10 GC-FI.% an D-Citronellal erreicht ist, auf 10 °C erwärmt und weitere 2 h bei dieser Temperatur gerührt. Anschließend wird zunächst die Reaktionslösung in einen Pufferbehälter überführt.

Die Reaktionslösung wird einer Bodenkolonne (15 Böden, DN 50) kontinuierlich mit einer Zulaufgeschwindigkeit von 300 g/h zugeführt. Toluol wird der Kolonne bei einem Kopfdruck von etwa 100 mbar an einem Seitenabzug auf dem 10. Boden im Verstärkungsteil entnommen, wobei die Sumpftemperatur etwa 120 °C und die Temperatur des Seitenabzugs und des Kolonnenkopfes 45 °C betragen. Am Kopf dieser Kolonne werden die Leichtsieder der Reaktionslösung ausgeschleust.

Ein Austrag des Sumpfproduktes der Bodenkolonne wird kontinuierlich (120 bis 140 g/h) einer Packungskolonne (DN 50 x 120 cm, Labor-Gewebe-Packung, Sulzer DX) mittig zugeführt. Unter kontinuierlicher Zugabe von Phenylcyclohexan (70 bis 90 g/h) in den Sumpf dieser Packungskolonne wird bei einer Sumpftemperatur von 110 °C und einem Kopfdruck von 10 mbar L-Isopulegol als Kopfprodukt abdestilliert. L-Isopulegol wird in einer Ausbeute von 1625 g und in einer Reinheit von 93 % isoliert.

### 2.b) Isolierung des Liganden (la₂-3) bei kontinuierlicher Fahrweise

Ein Austrag des Destillationssumpfes der Packungskolonne wird kontinuierlich (100 bis 120 g/h) einer auf 95 °C temperierten Mixer-Settler-Anlage bestehend aus zwei kaskadierten 250 ml-Rührbehältern und einem 150 ml-Phasenscheider zugeführt. Im ersten 250 ml-Rührbehälter wird der Austrag des Destillationssumpfes der Packungskolonne kontinuierlich mit einem Zulauf 2%iger Natriumhydroxid-Lösung (50 bis 60 g/h) versetzt. Ein Austrag (150 bis 180 g/h) der Mischphase aus dem ersten Rührbehälter wird über den weiteren 250 ml-Rührbehälter in den 150 ml-Phasenscheider überführt. Im Phasenscheider erfolgt die kontinuierliche Trennung der Phasen bei einer Temperatur von 90 bis 95 °C. Die Höhe der Phasentrennschicht wird dabei mit Hilfe von Leitfähigkeitsmessungen geregelt.

Der Austrag der organischen Phase aus dem Phasenscheider wird kontinuierlich (100 bis 120 g/h) in einem weiteren auf 40 bis 50 °C temperierten Rührbehälter aufgefangen und vor der Isolierung des Liganden (la₂-3) der Kristallisation überlassen. Ein Austrag der wässrigen Phase aus dem Phasenscheider wird kontinuierlich abgepumpt.

Der auskristallisierte Ligand (Ia₂-3) wird batchweise über einen Druckfilter bei einem Stickstoffdruck von 4 bar filtriert. Anschließend wird der Filterkuchen mit Phenylcyclohexan gewaschen. Der gewaschene Ligand (106 g; HPLC-Gew.-%: Ligand 77 %; Phenylcyclohexan 22 %) wird in Toluol gelöst und zur Katalysatorherstellung in Schritt 2.a) wiederverwendet. Das Filtrat (919 g; Gew.-% laut GC: Phenylcyclohexan 66 %; L-Isopulegol 5 %; Citronellol 6,1 %; Citronellsäureisopulegylester 4,3 %; Citronellsäurecitronellylester 3,6 %; Gew.-% laut HPLC: Ligand 3,1 %) wird in die unter 2.b) beschriebene Packungskolonne zurückgeführt.

## Patentansprüche

1. Verfahren zur Aufarbeitung eines Aluminium-haltigen Reaktionsprodukts aus der Herstellung von Isopulegol durch Cyclisierung von Citronellal, enthaltend
i) Isopulegol,
ii) wenigstens einen Liganden der Formel (I), 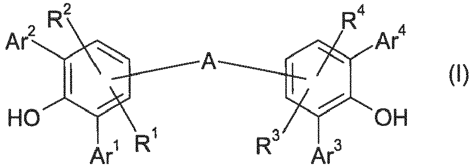
wobei
Ar¹, Ar², Ar³, Ar⁴ unabhängig voneinander ausgewählt sind unter C₆-C₁₅-Arylresten oder C₂-C₅-Heteroarylresten, die gegebenenfalls jeweils 1 bis 7 gleiche oder verschiedene Substituenten, ausgewählt unter C₁-C₆-Alkyl, C₁-C₆-Perfluoralkyl, C₁-C₆-Alkoxy, C₇-C₁₂-Aralkyl, Halogen, SiR^{5a}R^{6a}R^{7a}, gegebenenfalls substituiertem C₆-C₁₀-Aryl, NR^{8a}R^{9a}, SR^{10a}, NO₂ tragen können, bedeuten,
R¹, R², R³, R⁴ unabhängig voneinander ausgewählt sind unter Wasserstoff, C₁-C₆-Alkyl, C₁-C₆-Perfluoralkyl, C₁-C₆-Alkoxy, C₇-C₁₂-Aralkyl, Halogen, SiR^{5b}R^{6b}R^{7b}, gegebenenfalls substituiertem C₆-C₁₀-Aryl, NR^{8b}R^{9b}, SR^{10b}, NO₂ und wobei
R¹ oder R² und/oder R³ oder R⁴ gemeinsam mit A einen aromatischen oder nicht-aromatischen Cyclus bilden kann, bedeuten und
A für einen geradkettigen oder verzweigten und/oder cyclischen Kohlenwasserstoffrest mit 1 bis 25 C-Atomen steht, der gesättigt oder ein- oder mehrfach ungesättigt und/oder teilweise aromatisch sein kann und gegebenenfalls eines oder mehrere gleiche oder verschiedene Heteroatome, ausgewählt unter O, S, NR¹¹, und/oder eine oder mehrere gleiche oder verschiedene funktionelle Gruppen, ausgewählt unter den funktionellen Gruppen C(O), S(O), S(O)₂, aufweisen kann und gegebenenfalls einen oder mehrere gleiche oder verschiedene Substituenten, ausgewählt unter der Substituenten C₁-C₆-Alkyl, C₁-C₆-Perfluoralkyl, C₁-C₆-Alkoxy, C₁-C₁₀-Acyloxy, C₇-C₁₂-Aralky), Halogen, -SiR^{5c}R^{6c}R^{7c}, gegebenenfalls substituiertes C₆-C₁₀-Aryl, substituiertes oder unsubstituiertes C₂-C₁₀-Hetaryl, NR^{8c}R^{9c}, SR^{10c}, NO₂, C₁-C₁₂-Acyl, C₁-C₁₀-Carboxyl tragen kann, oder
für einen C₆-C₁₅-Arylrest oder einen C₂-C₁₅-Heteroarylrest steht, der gegebenenfalls jeweils 1 bis 5 Substituenten, ausgewählt unter C₁-C₆-Alkyl, C₁-C₆-Perfluoralkyl, C₁-C₆-Alkoxy, C₇-C₁₂-Aralkyl, Halogen, SiR^{5d}R^{6d}R^{7d}, substituiertes oder unsubstituiertes C₆-C₁₀-Aryl, NR^{8d}R^{9d}, SR^{10d}, NO₂ tragen können, oder
für eine funktionelle Gruppe oder ein Heteroatom ausgewählt aus der Gruppe -O-, -S-, -N(R¹¹)-, -S(O)-, -C(O)-, -S(O)₂-, -P(R¹¹)-, -(R¹¹)P(O)- und -Si(R¹²R¹³) steht,
wobei die Reste R^{5a}, R^{6a}, R^{7a}, R^{8a}, R^{9a}, R^{10a} bis R^{5d}, R^{6d}, R^{7d}, R^{8d}, R^{9d}, R^{10d}, R¹¹, R¹² und R¹³ jeweils unabhängig voneinander ausgewählt sind unter C₁-C₆-Alkyl, C₇-C₁₂-Aralkyl und/oder substituiertes oder unsubstituiertes C₆-C₁₀-Aryl und wobei die Reste R^{8a} und R^{9a}, R^{8b} und R^{9b}, R^{8c} und R^{9c}, R^{8d} und R^{9d} unabhängig voneinander jeweils gemeinsam auch einen cyclischen Kohlenwasserstoffrest mit 2 bis 8 Kohlenstoffatomen bilden können, der eines oder mehrere gleiche oder verschieden Heteroatome ausgewählt aus der Gruppe O, S, NR^{11a} aufweisen kann und R^{11a} die für R¹¹ angegebenen Bedeutungen haben kann,
in freier und/oder komplexgebundener Form,
bei dem man
a) das Reaktionsprodukt einer destillativen Auftrennung unter Erhalt eines an Isopulegol angereicherten Kopfprodukts und eines an Isopulegol abgereicherten Sumpfprodukts unterzieht,
b) das an Isopulegol abgereicherte Sumpfprodukt mit einer wässrigen Base unter Erhalt einer Aluminium-haltigen wässrigen Phase und einer die Hauptmenge der Liganden der Formel (I) enthaltenden organischen Phase in innigen Kontakt bringt,
c) den Liganden der Formel (I) aus der organischen Phase abtrennt.

2. Verfahren gemäß Anspruch 1, bei dem man den Ligand der Formel (I) in Schritt b) durch Kristallisation aus der organischen Phase abtrennt.

3. Verfahren gemäß einem der Ansprüche 1 oder 2, wobei das Reaktionsprodukt aus der Herstellung von Isopulegol durch Cyclisierung von Citronellal, zusätzlich ein niedriger siedendes Lösungsmittel (iii) enthält.

4. Verfahren gemäß einem der Ansprüche 1 bis 3, wobei das Reaktionsprodukt aus der Herstellung von Isopulegol durch Cyclisierung von Citronellal, zusätzlich einen Hilfsstoff (iv) enthält.

5. Verfahren gemäß Anspruch 4, wobei der Hilfsstoff ausgewählt ist unter organischen Säuren, Carbonsäureanhydriden, Aldehyden, Ketonen und Vinylethern.

6. Verfahren gemäß einem der Ansprüche 1 bis 5, wobei vor der destillativen Auftrennung in Schritt a) aus dem Reaktionsprodukt zunächst gegebenenfalls enthaltenes Lösungsmittel und/oder Hilfsstoffe aus der Cyclisierung abgetrennt werden.

7. Verfahren gemäß einem der Ansprüche 1 bis 6, wobei man das Reaktionsprodukt vor und/oder während der destillativen Auftrennung in Schritt a) mit einem Lösungsmittel versetzt, dessen Siedepunkt unter den Bedingungen der Destillation um wenigstens 10 °C höher liegt als der Siedepunkt des Isopulegols.

8. Verfahren gemäß einem der Ansprüche 1 bis 7, wobei wenigstens einer der Verfahrensschritte kontinuierlich betrieben wird.

9. Verfahren gemäß einem der Ansprüche 7 oder 8, wobei die Zugabe des höhersiedenden Lösungsmittels während Schritt a) erfolgt.

10. Verfahren gemäß einem der Ansprüche 1 bis 9, wobei man einen erwärmten Austrag des Sumpfprodukts aus Schritt a) mit einer erwärmten wässrigen Base in innigen Kontakt bringt und anschließend die Hauptmenge des Liganden aus der organischen Phase isoliert.

11. Verfahren gemäß einem der Ansprüche 1 bis 10, wobei der Ligand der Formel (I) ausgewählt ist unter Bis(diarylphenol)-Liganden der Formel (I.a) 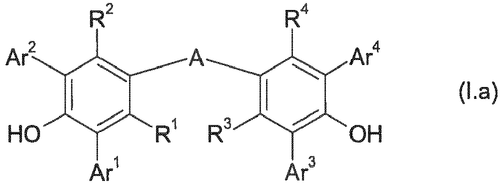 wobei
Ar¹, Ar², Ar³, Ar⁴, R¹, R², R³, R⁴ und A die in Anspruch 1 für Formel (I) angegebenen Bedeutungen besitzen.

12. Verfahren gemäß einem der Ansprüche 1 bis 11, wobei das Aluminium-haltige Reaktionsprodukt erhalten wird bei der Herstellung von Isopulegol der Formel (IV) 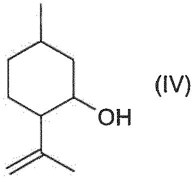 umfassend
α) die Cyclisierung von Citronellal der Formel (V) 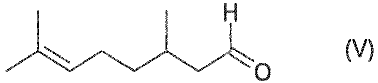 in Gegenwart eines Katalysators, der erhältlich ist durch Umsetzung eines Bis(diarylphenol)-Liganden der Formel (I), wie in den Ansprüchen 1 und/oder 11 definiert,
mit einer Aluminium-Verbindung der Formel (II),
(R¹⁴ )₃-pAlHp (II)
wobei
Al Aluminium bedeutet,
R¹⁴ einen verzweigten oder unverzweigten Alkylrest mit 1 bis 5 Kohlenstoffatomen und
p für 0 oder eine ganze Zahl von 1 bis 3 steht,
und/oder
mit einer Aluminium-Verbindung der Formel (III),
MAIH₄ (III)
wobei
Al Aluminium bedeutet und
M Lithium, Natrium oder Kalium bedeutet,
β) die Rückgewinnung des Bis(diarylphenol)-Liganden der Formel (I) nach erfolgter Umsetzung, indem man
a) das in Schritt α) erhaltene Reaktionsprodukt einer destillativen Auftrennung unter Erhalt eines an Isopulegol angereicherten Kopfprodukts und eines an Isopulegol abgereicherten Sumpfprodukts unterzieht,
b) das an Isopulegol abgereicherte Sumpfprodukts mit einer wässrigen Base unter Erhalt einer Aluminium-haltigen wässrigen Phase und einer die Hauptmenge der Liganden der Formel (I) enthaltenden organischen Phase in innigen Kontakt bringt und
c) den Liganden der Formel (I) aus der organischen Phase abtrennt.

13. Verfahren nach Anspruch 12, wobei die Aluminium-Verbindung ausgewählt ist unter Trimethyl- oder Triethylaluminium.

14. Verfahren gemäß einem der Ansprüche 12 oder 13 zur Herstellung von optisch aktivem Isopulegol der Formel (IV.a) 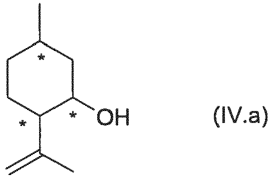 umfassend die Cyclisierung von optisch aktivem Citronellal der Formel (V.a) 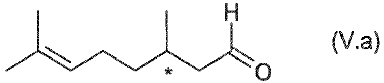 wobei (*) jeweils ein asymmetrisches Kohlenstoffatom bezeichnet.

15. Verfahren zur Herstellung von Menthol umfassend die Schritte:
A) Herstellung von Isopulegol der Formel (IV) gemäß einem der Ansprüche 12 bis 14 und
B) Hydrierung der ethylenischen Doppelbindung des so erhaltenen Isopulegols.

## Claims

1. A method for working up an aluminum-containing reaction product from the production of isopulegol by cyclizing citronellal, comprising
i) isopulegol,
ii) at least one ligand of the formula (I), 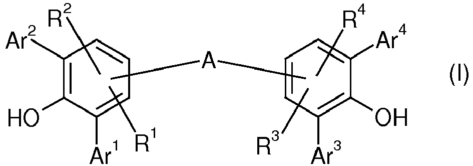
where
Ar¹, Ar², Ar³, Ar⁴, independently of one another, are chosen from C₆-C₁₅-aryl radicals or C₂-C₁₅-heteroaryl radicals, which, if appropriate, can in each case carry 1 to 7 identical or different substituents chosen from C₁-C₆-alkyl, C₁-C₆-perfluoroalkyl, C₁-C₆-alkoxy, C₇-C₁₂-aralkyl, halogen, SiR^{5a}R^{6a}R^{7a}, optionally substituted C₆-C₁₀-aryl, NR^{8a}R^{9a}, SR^{10a}, NO₂,
R¹, R², R³, R⁴, independently of one another, are chosen from hydrogen, C₁-C₆-alkyl, C₁-C₆-perfluoroalkyl, C₁-C₆-alkoxy, C₇-C₁₂-aralkyl, halogen, SiR^{5b}R^{6b}R^{7b}, optionally substituted C₆-C₁₀-aryl, NR^{8b}R^{9b}, SR^{10b}, NO₂ and where
R¹ or R² and/or R³ or R⁴, together with A, can form an aromatic or nonaromatic cycle, and
A is a straight-chain or branched and/or cyclic hydrocarbon radical having 1 to 25 carbon atoms which may be saturated or mono- or polyunsaturated and/or partially aromatic and can, if appropriate, have one or more identical or different heteroatoms chosen from 0, S, NR¹¹, and/or one or more identical or different functional groups chosen from the functional groups C(O), S(O), S(O)₂ and can, if appropriate, carry one or more identical or different substituents chosen from the substituents C₁-C₆-alkyl, C₁-C₆-perfluoroalkyl, C₁-C₆-alkoxy, C₁-C₁₀-acyloxy, C₇-C₁₂-aralkyl, halogen, -SiR^{5c}R^{6c}R^{7c}, optionally substituted C₆-C₁₀-aryl, substituted or unsubstituted C₂-C₁₀-hetaryl, NR^{8c}R^{9c}, SR^{10c}, NO₂, C₁-C₁₂-acyl, C₁-C₁₀-carboxyl, or
is a C₆-C₁₅-aryl radical or a C₂-C₁₅-heteroaryl radical which can, if appropriate, in each case carry 1 to 5 substituents chosen from C₁-C₆-alkyl, C₁-C₆-perfluoroalkyl, C₁-C₆-alkoxy, C₇-C₁₂-aralkyl, halogen, SiR^{5d}R^{6d}R^{7d}, substituted or unsubstituted C₆-C₁₀-aryl, NR^{8d}R^{9d}, SR^{10d}, NO₂, or
is a functional group or a heteroatom chosen from the group -O-, -S-, -N(R¹¹)-, - S(O)-, -C(O)-, -S(O)₂-, -P(R¹¹) -, -(R¹¹)P(O)- and -Si (R¹²R¹³),
where the radicals R^{5a}, R^{6a}, R^{7a}, R^{8a}, R^{9a}, R^{10a} to R^{5d}, R^{6d}, R^{7d}, R^{8d}, R^{9d}, R^{10d}, R¹¹, R¹² and R¹³ are in each case independently of one another chosen from C₁-C₆-alkyl, C₇-C₁₂-aralkyl and/or substituted or unsubstituted C₆-C₁₀-aryl and where the radicals R^{8a} and R^{9a}, R^{8b} and R^{9b}, R^{8c} and R^{9c}, R^{8d} and R^{9d}, independently of one another, can in each case together also form a cyclic hydrocarbon radical having 2 to 8 carbon atoms which can have one or more identical or different heteroatoms chosen from the group 0, S, NR^{11a}, and R^{11a} can have the meanings given for R¹¹,
in free and/or complex-bound form,
in which
a) the reaction product is subjected to distillative separation to obtain an isopulegol-enriched top product and an isopulegol-depleted bottom product,
b) the isopulegol-depleted bottom product is brought into close contact with an aqueous base to give an aluminum-containing aqueous phase and an organic phase comprising the majority of the ligands of the formula (I),
c) the ligands of the formula (I) are separated off from the organic phase.

2. The method according to claim 1, in which the ligand of the formula (I) is separated off from the organic phase in step b) by crystallization.

3. The method according to one of claims 1 or 2, where the reaction product from the production of isopulegol by cyclizing citronellal additionally comprises a lower-boiling solvent (iii).

4. The method according to one of claims 1 to 3, where the reaction product from the production of isopulegol by cyclizing citronellal additionally comprises an auxiliary (iv).

5. The method according to claim 4, where the auxiliary is chosen from organic acids, carboxylic anhydrides, aldehydes, ketones and vinyl ethers.

6. The method according to one of claims 1 to 5, where, prior to the distillative separation in step a), any solvent present and/or auxiliaries from the cyclization are firstly separated off from the reaction product.

7. The method according to one of claims 1 to 6, where, before and/or during the distillative separation in step a), the reaction product is admixed with a solvent whose boiling point, under the distillation conditions, is at least 10°C higher than the boiling point of isopulegol.

8. The method according to one of claims 1 to 7, where at least one of the process steps is operated continuously.

9. The method according to one of claims 7 or 8, where the addition of the higher-boiling solvent takes place during step a).

10. The method according to one of claims 1 to 9, where a heated discharge of the bottom product from step a) is brought into close contact with a heated aqueous base and then the majority of the ligand is isolated from the organic phase.

11. The method according to one of claims 1 to 10, where the ligand of the formula (I) is chosen from bis(diarylphenol) ligands of the formula (I.a) 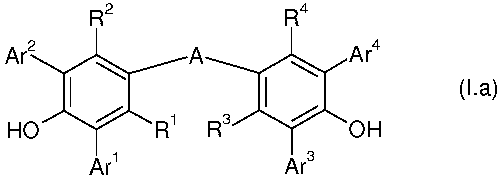 where
Ar¹, Ar², Ar³, Ar⁴, R¹, R², R³, R⁴ and A have the meanings given in claim 1 for formula (I).

12. The method according to one of claims 1 to 11, where the aluminum-containing reaction product is obtained during the production of isopulegol of the formula (IV) 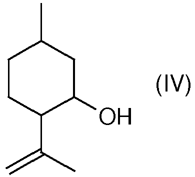 comprising
α) the cyclization of citronellal of the formula (V) 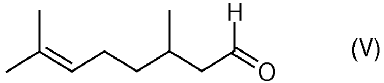 in the presence of a catalyst which is obtainable by reacting a bis(diarylphenol) ligand of the formula (I) as defined in claims 1 and/or 11,
with an aluminum compound of the formula (II),
(R¹⁴)₃₋ₚAlHₚ (II)
where
Al is aluminum,
R¹⁴ is a branched or unbranched alkyl radical having 1 to 5 carbon atoms and
p is 0 or an integer from 1 to 3,
and/or
with an aluminum compound of the formula (III),
MAlH₄ (III)
where
Al is aluminum and
M is lithium, sodium or potassium,
β) the recovery of the bis (diarylphenol) ligand of the formula (I) after the reaction has taken place by
a) subjecting the reaction product obtained in step α) to distillative separation to obtain an isopulegol-enriched top product and an isopulegol-depleted bottom product,
b) bringing the isopulegol-depleted bottom product into close contact with an aqueous base to give an aluminum-containing aqueous phase and an organic phase comprising the majority of the ligands of the formula (I) and
c) separating off the ligand of the formula (I) from the organic phase.

13. The method according to claim 12, where the aluminum compound is chosen from trimethyl- or triethylaluminum.

14. The method according to either of claims 12 and 13 for producing optically active isopulegol of the formula (IV.a) 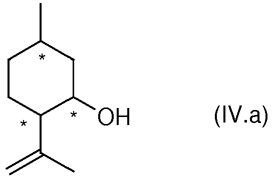 comprising the cyclization of optically active citronellal of the formula (V.a) 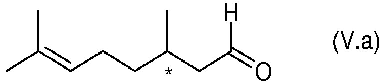 where (*) in each case refers to an asymmetric carbon atom.

15. A method for producing menthol comprising the steps:
A) production of isopulegol of the formula (IV) according to one of claims 12 to 14 and
B) hydrogenation of the ethylenic double bond of the isopulegol obtained in this way.

## Revendications

1. Procédé de traitement d'un produit de réaction contenant de l'aluminium issu de la fabrication d'isopulégol par cyclisation de citronellal, contenant
i) de l'isopulégol,
ii) au moins un ligand de formule (I) 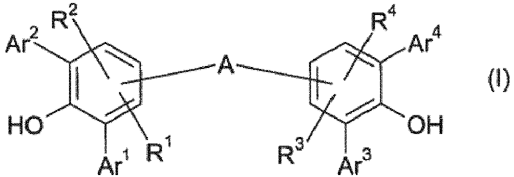 dans laquelle
Ar¹, Ar², Ar³, Ar⁴ sont choisis indépendamment les uns des autres parmi les radicaux aryle en C₆-C₁₅ ou les radicaux hétéroaryle en C₂-C₁₅, qui peuvent éventuellement chacun porter 1 à 7 substituants identiques ou différents, choisis parmi alkyle en C₁-C₆, perfluoroalkyle en C₁-C₆, alcoxy en C₁-C₆, aralkyle en C₇-C₁₂, halogène, SiR^{5a}R^{6a}R^{7a}, aryle en C₆-C₁₀ éventuellement substitué, NR^{8a}R^{9a}, SR^{10a}, NO₂,
R¹, R², R³, R⁴ sont choisis indépendamment les uns des autres parmi hydrogène, alkyle en C₁-C₆, perfluoroalkyle en C₁-C₆, alcoxy en C₁-C₆, aralkyle en C₇-C₁₂, halogène, SiR^{5b}R^{6b}R^{7b}, aryle en C₆-C₁₀ éventuellement substitué, NR^{8b}R^{9b}, SR^{10b}, NO₂,
R¹ ou R² et/ou R³ ou R⁴ pouvant former conjointement avec A un cycle aromatique ou non aromatique, et
A représente un radical hydrocarboné linéaire ou ramifié et/ou cyclique de 1 à 25 atomes C, qui peut être saturé ou mono- ou polyinsaturé et/ou partiellement aromatique, et qui peut éventuellement comprendre un ou plusieurs hétéroatomes identiques ou différents choisis parmi 0, S, NR¹¹, et/ou un ou plusieurs groupes fonctionnels identiques ou différents, choisis parmi les groupes fonctionnels C(O), S(O), S(O)₂, et qui peut éventuellement porter un ou plusieurs substituants identiques ou différents, choisis parmi les substituants alkyle en C₁-C₆, perfluoroalkyle en C₁-C₆, alcoxy en C₁-C₆, acyloxy en C₁-C₁₀, aralkyle en C₇-C₁₂, halogène,-SiR^{5c}R^{6c}R^{7c}, aryle en C₆-C₁₀ éventuellement substitué, hétaryle en C₂-C₁₀ substitué ou non substitué, NR^{8c}R^{9c}, SR^{10c}, NO₂, acyle en C₁-C₁₂, carboxyle en C₁-C₁₀, ou
un radical aryle en C₆-C₁₅ ou un radical hétéroaryle en C₂-C₁₅, qui peuvent éventuellement chacun porter 1 à 5 substituants, choisis parmi alkyle en C₁-C₆, perfluoroalkyle en C₁-C₆, alcoxy en C₁-C₆, aralkyle en C₇-C₁₂, halogène, SiR^{5d}R^{6d}R^{7d}, aryle en C₆-C₁₀ substitué ou non substitué, NR^{8d}R^{9d}, SR^{10d}, NO₂, ou
un groupe fonctionnel ou un hétéroatome choisi dans le groupe constitué par -O-, -S-, -N(R¹¹) -, -S(O)-, -C(O)-, -S(O)₂-, -P(R¹¹)-, -(R¹¹)P(O)-et -Si(R¹²R¹³)),
les radicaux R^{5a}, R^{6a}, R^{7a}, R^{8a}, R^{9a}, R^{10a} à R^{5d}, R^{6d}, R^{7d}, R^{8d}, R^{9d}, R^{10d}, R¹¹, R¹² et R¹³ étant chacun choisis indépendamment les uns des autres parmi alkyle en C₁-C₆, aralkyle en C₇-C₁₂, et/ou aryle en C₆-C₁₀ substitué ou non substitué, et les radicaux R^{8a} et R^{9a}, R^{8b} et R^{9b}, R^{8c} et R^{9c}, R^{8d} et R^{9d} pouvant chacun indépendamment les uns des autres également former ensemble un radical hydrocarboné cyclique de 2 à 8 atomes de carbone, qui peut comprendre un ou plusieurs hétéroatomes identiques ou différents choisis dans le groupe constitué par O, S, NR^{11a}, et R^{11a} pouvant avoir les significations indiquées pour R¹¹, sous forme libre et/ou complexée,
selon lequel
a) le produit de réaction est soumis à une séparation par distillation pour obtenir un produit de tête enrichi en isopulégol et un produit de fond appauvri en isopulégol,
b) le produit de fond appauvri en isopulégol est mis en contact intime avec une base aqueuse pour obtenir une phase aqueuse contenant de l'aluminium et une phase organique contenant la quantité principale des ligands de formule (I),
c) les ligands de formule (I) sont séparés de la phase organique.

2. Procédé selon la revendication 1, selon lequel le ligand de formule (I) est séparé de la phase organique par cristallisation à l'étape b).

3. Procédé selon l'une quelconque des revendications 1 ou 2, dans lequel le produit de réaction issu de la fabrication d'isopulégol par cyclisation de citronellal contient en outre un solvant de plus faible point d'ébullition (iii).

4. Procédé selon l'une quelconque des revendications 1 à 3, dans lequel le produit de réaction issu de la fabrication d'isopulégol par cyclisation de citronellal contient en outre un adjuvant (iv).

5. Procédé selon la revendication 4, dans lequel l'adjuvant est choisi parmi les acides organiques, les anhydrides d'acides carboxyliques, les aldéhydes, les cétones et les éthers de vinyle.

6. Procédé selon l'une quelconque des revendications 1 à 5, dans lequel, avant la séparation par distillation à l'étape a), les solvants et/ou les adjuvants issus de la cyclisation éventuellement contenus sont tout d'abord séparés du produit de réaction.

7. Procédé selon l'une quelconque des revendications 1 à 6, dans lequel le produit de réaction est mélangé avant et/ou pendant la séparation par distillation à l'étape a) avec un solvant dont le point d'ébullition dans les conditions de la distillation est supérieur d'au moins 10 °C au point d'ébullition de l'isopulégol.

8. Procédé selon l'une quelconque des revendications 1 à 7, dans lequel au moins une des étapes de procédé est réalisée en continu.

9. Procédé selon l'une quelconque des revendications 7 ou 8, dans lequel l'ajout du solvant de point d'ébullition plus élevé a lieu pendant l'étape a).

10. Procédé selon l'une quelconque des revendications 1 à 9, dans lequel un déchargement chauffé du produit de fond de l'étape a) est mis en contact intime avec une base aqueuse chauffée, puis la quantité principale du ligand est isolée de la phase organique.

11. Procédé selon l'une quelconque des revendications 1 à 10, dans lequel le ligand de formule (I) est choisi parmi les ligands bis(diarylphénol) de formule (I.a) 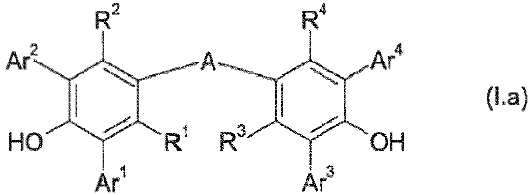 dans laquelle
Ar¹, Ar², Ar³, Ar⁴, R¹, R², R³, R⁴ et A ont les significations indiquées dans la revendication 1 pour la formule (I).

12. Procédé selon l'une quelconque des revendications 1 à 11, dans lequel le produit de réaction contenant de l'aluminium est obtenu lors de la fabrication d'isopulégol de formule (IV) 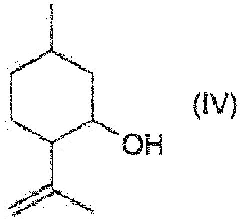 comprenant
α) la cyclisation de citronellal de formule (V) 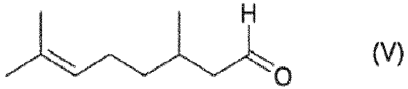 en présence d'un catalyseur qui peut être obtenu par mise en réaction d'un ligand bis(diarylphénol) de formule (I), tel que défini dans les revendications 1 et/ou 11,
avec un composé d'aluminium de formule (II)
(R¹⁴)₃₋ₚAlHₚ (II)
dans laquelle
Al signifie aluminium,
R¹⁴ représente un radical alkyle ramifié ou non ramifié de 1 à 5 atomes de carbone, et
p représente 0 ou un nombre entier de 1 à 3, et/ou
avec un composé d'aluminium de formule (III)
MAlH₄ (III)
dans laquelle
Al représente aluminium et
M représente lithium, sodium ou potassium,
β) la récupération du ligand bis(diarylphénol) de formule (I) après la réaction par
a) soumission du produit de réaction obtenu à l'étape α) à une séparation par distillation pour obtenir un produit de tête enrichi en isopulégol et un produit de fond appauvri en isopulégol,
b) mise en contact intime du produit de fond appauvri en isopulégol avec une base aqueuse pour obtenir une phase aqueuse contenant de l'aluminium et une phase organique contenant la quantité principale des ligands de formule (I), et
c) séparation des ligands de formule (I) de la phase organique.

13. Procédé selon la revendication 12, dans lequel le composé d'aluminium est choisi parmi le triméthyl- ou triéthylaluminium.

14. Procédé selon l'une quelconque des revendications 12 ou 13 pour la fabrication d'isopulégol optiquement actif de formule (IV.a) 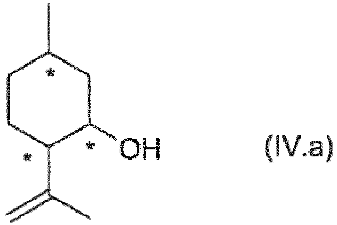 comprenant la cyclisation de citronellal optiquement actif de formule (V.a) 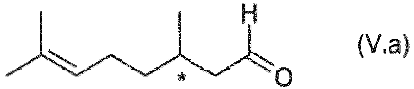 (*) désignant à chaque fois un atome de carbone asymétrique.

15. Procédé de fabrication de menthol comprenant les étapes suivantes:
A) la fabrication d'isopulégol de formule (IV) selon l'une quelconque des revendications 12 à 14, et
B) l'hydrogénation de la double liaison éthylénique de l'isopulégol ainsi obtenu.
